# FASCICULE DE BREVET EUROPEEN

(11) **EP 0 470 873 B1**
(45) Date de publication et mention de la délivrance du brevet: **07.12.1994**
(21) Numéro de dépôt: 91401843.7
(22) Date de dépôt: 04.07.1991
(51) Int. Cl.: C07C 331/10, C07D 251/38, C08F 28/00

(54) **Nouveaux composés (méth)acryliques porteurs de fonction thiocyanate, leur procédé de préparation et leur application à l'obtention de nouveaux polymères**
Thiocyanatgruppe enthaltende (Meth)acrylsäureester, Verfahren zu ihrer Herstellung und ihre Verwendung bei der Herstellung von neuen Polymeren
(Meth)acrylic compounds containing the thiocyanate group, process for their preparation and their use in the preparation of new polymers

(30) Priorité: 09.07.1990 FR 9008698
(43) Date de publication de la demande: 12.02.1992
(73) Titulaire: ELF ATOCHEM S.A., 92800 Puteaux (FR)
(72) Inventeur: Berthe, Marie-Christine, F-54500 Vandoeuvre Les Nancy (FR); Caubere, Paul, F-54000 Nancy (FR); Fort, Yves, F-54500 Vandoeuvre Les Nancy (FR)

(56) Documents cités:
- CHEMICAL ABSTRACTS, vol. 83, 1975, page 428, abrégé no. 199643d, Columbus, Ohio, US; V.E. SOLODKIN: "Luminescence characteristics of electroluminescent layers based on binders with thiocyanate groups"
- CHEMICAL ABSTRACTS, vol. 81, 1974, page 389, abrégé no. 83548f, Columbus, Ohio, US; & SU-A-411 113
- CHEMICAL ABSTRACTS, 9TH COLLECTIVE INDEX 1972-1976, page C7H9NO2S, Columbus, Ohio, US; "2-Propenoic acid, 2-cyano-3-(methylthio)-ethyl ester, 2-methyl-2-thiocyanatoethyl ester, homopolymer"
- CHEMICAL ABSTRACTS, REGISTRY NUMBER INDEX, Columbus, Ohio, US, 76392-31-9, 53397-61-8

## Description

La présente invention se rapporte à la synthèse de nouveaux composés acryliques et méthacryliques porteurs d'au moins une fonction thiocyanate ainsi qu'à l'application de ces composés à la préparation de nouveaux polymères et copolymères.

On connaît déjà dans la littérature scientifique et industrielle de nombreux composés acryliques et méthacryliques porteurs de fonctions telles que halogène, hydroxyle, thiol, époxyde, etc. Chacune de ces familles de composés a déjà trouvé dans différentes industries, en raison de la facilité de polymérisation de la double liaison acrylique, des applications variées. Jusqu'à ce jour toutefois, la littérature scientifique et industrielle n'a pas donné d'exemples de composés acryliques et méthacryliques porteurs de fonction thiocyanate. En raison des particularités de comportement chimique que l'on peut attendre de la présence d'une telle fonction dans un composé acrylique ou méthacrylique, le but visé par la présente invention consiste donc à explorer ce nouveau domaine de la chimie et à déterminer les conditions de synthèse de tels composés.

Un premier objet de la présente invention concerne donc des composés acryliques et méthacryliques choisis parmi ceux de formule : 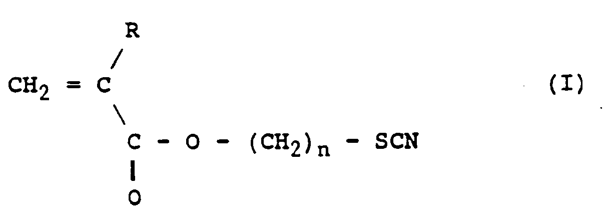
et ceux de formule 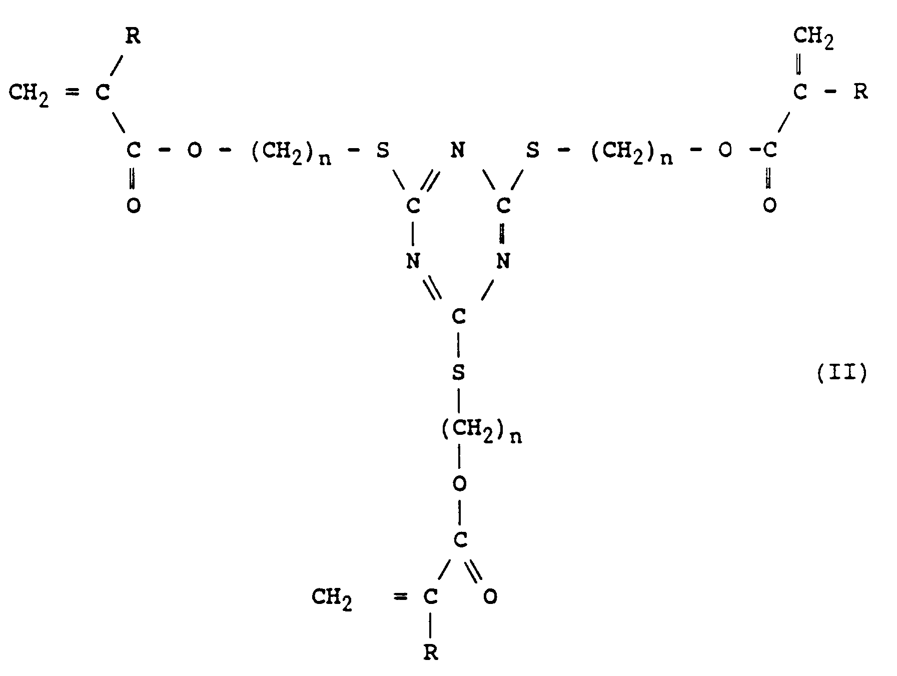
dans lesquelles :
- n est un nombre entier allant de 2 à 20, et
- R est choisi parmi l'atome d'hydrogène, le radical méthyle et les radicaux CHOHR' dans lesquels R' est choisi parmi les radicaux alkyles linéaires ou ramifiés le cas échéant porteurs de substituants hydroxyles, alcoxy ou esters, les radicaux alcényles linéaires, ramifiés ou cycliques, les radicaux aryles le cas échéant porteurs de substituants halogénés, nitrés ou alcoxy, les radicaux hétérocycliques insaturés, les radicaux alkylaryles et arylalkyles,
R ne pouvant représenter méthyle dans la formule (I) si simultanément n vaut 2 ou 4.

Comme exemples de radicaux R' dans les composés de formules (I) et (II) selon l'invention, lorsque R est égal à CHOHR', on peut citer notamment les radicaux méthyle, éthyle, n-propyle, isopropyle, isobutyle, tertiobutyle, n-pentyle, n-hexyle, n-heptyle, n-nonyle, trichlorométhyle, chloro-3-propyle, propényl-1, méthyl-2-propényl-2, cyclohexényle, phényle, benzyle, phényl-2-éthyle, parachlorophényle, paratoluyle, paraméthoxyphényle, paranitrophényle, orthochlorophényle, styryle, méthoxyméthyle, acétyl-2-éthyle, pyridyl-2, pyridyl-3, pyridyl-4, quinolyl-4, furyl-2, méthyl-4-furyl-2, (furyl-2)-2-vinyl-1, oxo-2-propyl-1, méthane-sulfonate-2 d'éthyl-1, benzyloxy-1-éthyl-1, benzoyloxy-3-propyl-1, benzoyloxy-3-butyl-1, benzyloxyméthyl, (méthyl-2-dioxolanne-1,3)-2-éthyl-1, (tétrahydropyrannyl oxy-2)-3-propyl-1, acétyloxy-3-pyvaloyloxy-3-propyl-1, et 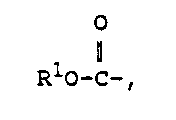
R¹ étant un radical alkyle ayant de 1 à 18 atomes de carbone.

Un second objet de la présente invention consiste en un procédé de préparation des composés acryliques et méthacryliques de formules (I) et (II). Quoique tous ces composés aient en commun pour leur préparation une étape mettant en jeu un sel de thiocyanate et un acrylate ou méthacrylate halogéné, leur synthèse présente toutefois des particularités selon que R est choisi parmi l'atome d'hydrogène et le radical méthyle ou bien selon que R est choisi parmi les radicaux CHOHR' tels que précédemment définis. C'est pourquoi le procédé de préparation selon l'invention sera maintenant décrit par référence à chacune des familles de composés selon l'invention.

La préparation des composés acryliques et méthacryliques de formule (I) dans laquelle R est choisi parmi l'atome d'hydrogène et le radical méthyle et n est un nombre entier allant de 2 à 20, s'effectue en faisant réagir un acrylate ou méthacrylate d'halogéno-n-alkyle avec un sel de thiocyanate dans un solvant organique et en présence d'une quantité efficace d'au moins un agent de transfert de phase. Le (méth)acrylate d'halogéno-n-alkyle qui est mis à réagir selon ce procédé a pour formule générale : 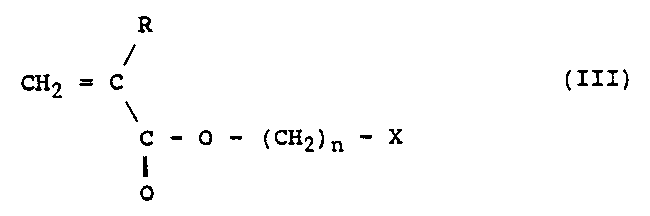
dans laquelle R et n sont tels que définis ci-dessus et X est un atome d'halogène. X est choisi de préférence parmi le chlore et le brome, mais peut être aussi l'iode. Les (méth)acrylates d'halogéno-n-alkyle de formule (III) peuvent être eux-mêmes obtenus par estérification du chlorure d'acide (méth)acrylique par un halogéno-alcool de formule HO-(CH₂)ₙ-X, cette réaction s'effectuant dans un solvant organique tel que le chloroforme, le cas échéant en présence d'un capteur d'acide chlorhydrique tel qu'une amine tertiaire.

Comme sel de thiocyanate pouvant être mis à réagir avec le (méth)acrylate d'halogéno-n-alkyle de formule (III) on peut citer notamment les thiocyanates de métal alcalin tel que le sodium et le potassium et le thiocyanate d'ammonium. Comme solvant organique pouvant convenir à cette réaction, on peut citer des cétones telles que l'acétone, la méthylisobutylcétone et la méthyléthylcétone, des amides tels que le diméthylformamide, le formamide et le N,N-diméthylacétamide, des hydrocarbures cyclaniques ou aromatiques tels que le cyclohexane, le méthylcyclohexane, le benzène et le toluène, des alcools tels que l'éthanol, des nitriles tels que l'acétonitrile et le benzonitrile, ainsi que des solvants chlorés tels que le chlorobenzène, l'orthodichlorobenzène, le dichloro-1,2-éthane et le tétrachlorure de carbone.

Le solvant organique est utilisé dans une proportion qui peut varier en fonction du (méth)acrylate d'halogéno-n-alkyle d'une part, de la nature du solvant d'autre part. Toutefois les plus usuelles sont généralement comprises entre 0,5 et 2,5 moles de (méth)acrylate de formule (III) par litre de solvant organique.

La température réactionnelle dépend évidemment de la nature du solvant organique choisi, étant donné qu'on opère le plus souvent à la température de reflux dudit solvant. Ainsi la température réactionnelle est le plus souvent choisie entre 50°C et 120°C environ. Enfin la réaction selon l'invention est effectuée en présence d'au moins un agent de transfert de phase qui peut être choisi notamment parmi :
- les sels d'ammonium quaternaires tels que les chlorure, bromure et iodure de tétra-n-butylammonium, l'hydrogénosulfate de tétra-n-butylammonium, le bromure de triméthylphénylammonium, le chlorure de méthyltricaprylylammonium, les chlorures de triméthylbenzyl- et triéthylbenzylammonium, les chlorure et bromure de tétraméthylammonium, l'iodure de tétraéthylammonium, et
- les sels de phosphonium quaternaires tels que le bromure de méthyltriphénylphosphonium, le bromure de tétra-n-butylphosphonium et l'iodure de triphényliodophosphonium.

La quantité efficace d'agent de transfert de phase dépend évidemment de la nature de l'agent de transfert choisi ainsi que du substrat - (méth)acrylate de formule (III) - concerné par la réaction. Toutefois elle est généralement comprise entre 0,04 et 0,4 mole d'agent de transfert pour 1 mole de substrat.

Dans le procédé selon l'invention, on utilise généralement une proportion de 1 à 2 moles environ de sel de thiocyanate pour 1 mole de (méth)acrylate de formule (III). Enfin le procédé peut être mis en oeuvre en présence d'un halogénure, de préférence un bromure ou un iodure, de métal alcalin, de préférence le sodium ou le potassium. Cet halogénure de métal alcalin, permettant de réaliser in situ un échange d'halogène pour obtenir un substrat plus réactif, peut être utilisé dans une proportion allant jusqu'à 1 mole pour 1 mole de (méth)acrylate de formule (III).

Quoique la pression atmosphérique soit généralement satisfaisante, le procédé selon l'invention peut également être mis en oeuvre sous pression réduite, par exemple entre 0,05 et 1 bar environ.

Enfin, la réaction selon l'invention peut être effectuée en présence d'une quantité efficace d'au moins un inhibiteur de polymérisation.

Cet inhibiteur est utilisé par exemple à raison de 0,05% à 0,5% en poids sur la base de (méth)acrylate. Comme exemples d'inhibiteurs de polymérisation utilisables, on peut citer notamment la phénothiazine, l'éther méthylique de l'hydroquinone, la N,N-diéthylhydroxylamine, le nitrobenzène, le di-tertiobutylcatéchol, l'hydroquinone, le p-anilinophénol, le phosphite de di-(2-éthylhexyl)-octylphényl et leurs mélanges en toutes proportions.

La préparation des composés acryliques de formule (I) dans laquelle R est choisi parmi les radicaux CHOHR' peut s'effectuer en faisant réagir un composé acrylique de formule (I) dans laquelle R est l'atome d'hydrogène - ce composé étant obtenu tel que décrit précédemment - avec un aldéhyde en présence d'une quantité efficace d'au moins un catalyseur de fonctionnalisation. Comme catalyseur de fonctionnalisation convenant bien à la réaction avec un aldéhyde on peut citer notamment des bases relativement fortes telles que des amines tertiaires cycliques ayant au moins un atome d'azote commun à trois cycles, décrites dans le brevet US-A-3 743 669, par exemple le diazabicyclo-[2,2,2]-octane, la quinuclidine, et l'α-quinuclidinol. Une quantité efficace de catalyseur de fonctionnalisation dépend évidemment de la nature de ce dernier, mais aussi de l'acrylate de formule (I) (R = H) et de l'aldéhyde. Elle est généralement comprise entre 0,1 et 10% environ, de préférence entre 1 et 6% environ en moles par rapport à la somme des réactants en présence - acrylate de formule (I) (R = H) et aldéhyde.

L'aldéhyde avec lequel est mis à réagir l'acrylate de formule (I) (R = H) a pour formule générale R'CHO, dans laquelle R' a la signification déjà mentionnée ci-dessus. Comme exemple de tels aldéhydes on peut citer notamment l'acétaldéhyde, le n-butyraldéhyde, le phénylacétaldéhyde, le benzaldéhyde, le crotonaldéhyde , le m-éthylphényacétaldéhyde, le m-chlorobenzaldéhyde, le p-nitrophénylacétaldéhyde, le m-carbométhoxybenzaldéhyde, le p-méthoxybenzaldéhyde, le formaldéhyde, le propionaldéhyde, l'isobutyraldéhyde, le tertiobutyraldéhyde, le n-pentaldéhyde, le n-hexaldéhyde, le n-heptaldéhyde, le n-nonaldéhyde, le chloro-4-butyraldéhyde, le p-chlorobenzaldéhyde, l'o-chlorobenzaldéhyde, le cyclohexène-4-aldéhyde, le furfuraldéhyde, le méthyl-4-furfuraldéhyde,..

Les réactants - aldéhyde et acrylate de formule (I) dans laquelle R est hydrogène - sont généralement utilisés dans une proportion de 0,5 à 2 moles d'aldéhyde pour 1 mole d'acrylate. La température à laquelle est effectuée la réaction est généralement comprise entre 0°C et 150°C environ. La réaction entre l'aldéhyde et l'acrylate peut être effectuée en outre en présence d'au moins un activateur électrophile tel qu'un sel de lithium, en particulier un halogénure de lithium comme le chlorure de lithium. Celui-ci peut être utilisé à raison de jusqu'à 0,1 % en moles par rapport à la somme des réactants.

Enfin, cette réaction selon l'invention peut être effectuée en présence d'une quantité efficace d'au moins un inhibiteur de polymérisation.

Cet inhibiteur est utilisé par exemple à raison de 0,05% à 0,5% en poids sur la base de (méth)acrylate. Comme exemples d'inhibiteurs de polymérisation utilisables, on peut citer notamment la phénothiazine, l'éther méthylique de l'hydroquinone, la N,N-diéthylhydroxylamine, le nitrobenzène, le di-tertiobutylcatéchol, l'hydroquinone, le p-anilinophénol, le phosphite de di-(2-éthylhexyl)-octylphényl et leurs mélanges en toutes proportions.

La préparation des composés acryliques de formule (I) dans laquelle R est choisi parmi les radicaux CHOHR' peut aussi s'effectuer en faisant d'abord réagir un acrylate d'halogéno-n-alkyle de formule (III) avec un aldéhyde de formule R'CHO en présence d'une quantité efficace d'au moins un catalyseur de fonctionnalisation pour obtenir un intermédiaire de formule : 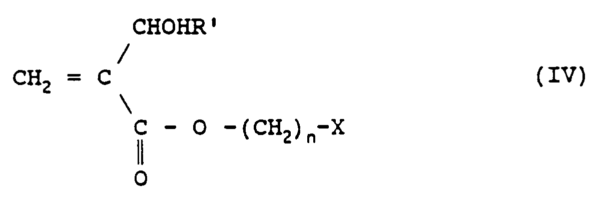
puis dans une seconde étape à faire réagir ledit intermédiaire avec un sel de thiocyanate dans un solvant organique et en présence d'une quantité efficace d'au moins un agent de transfert de phase. Les conditions opératoires de la première étape de ce procédé, notamment la nature du catalyseur de fonctionnalisation, la température réactionnelle et les proportions des divers réactants, sont analogues à celles décrites ci-dessus à propos du premier procédé de préparation. Les conditions opératoires de la seconde étape du procédé, notamment la nature du solvant organique, la nature de l'agent de transfert de phase, la température réactionnelle et les proportions des divers réactants, sont analogues à celles décrites précédemment a propos des composés acryliques et méthacryliques de formule (I) dans laquelle R est choisi parmi l'atome d'hydrogène et le radical méthyle. Les composés acryliques de formule (IV) ci-dessus constituant de nouveaux intermédiaires de synthèse. La première étape de ce procédé pourra le cas échéant être effectuée en présence d'une quantité efficace d'au moins un activateur électrophile tel qu'un sel de lithium, la notion de quantité efficace étant analogue à celle indiquée précédemment pour l'emploi de ces composés. Cette seconde méthode de préparation est plus particulièrement applicable aux composés dans lesquels n est un nombre entier allant de 2 à 4.

La préparation des composés acryliques et méthacryliques de formule (II), c'est-à-dire les trimères des composés acryliques et méthacryliques de formule (I), peut être effectuée par réaction desdits composés de formule (I) avec au moins un hydrure de métal alcalin comme le lithium ou le sodium, en présence d'un solvant organique. Comme solvant organique on peut utiliser notamment un hydrocarbure aromatique tel que le benzène, le toluène, les xylènes ou bien aliphatique tel que l'heptane. Cette réaction est effectuée à une température généralement comprise entre 50°C et 120°C environ, selon la nature du solvant choisi. La durée de la réaction est relativement longue et généralement comprise entre 2 et 80 heures environ, selon la température réactionnelle. Cette réaction est généralement incomplète et conduit, à l'issue de la durée mentionnée précédemment, à un mélange du composé de formule (I) et de son trimère de formule (II), mélange à partir duquel il est possible d'isoler, puis de purifier, le composé acrylique ou méthacrylique de formule (II).

Enfin un troisième et dernier objet de la présente invention consiste en l'application des nouveaux composés acryliques et méthacryliques décrits précédemment à la constitution de nouveaux polymères et copolymères. Plus précisément la présente invention concerne des polymères et copolymères comprenant au moins un motif dérivé d'au moins un composé acrylique ou méthacrylique de formule (I) et/ou (II), R, R' et n étant tels que définis ci-dessus, R ne pouvant représenter méthyle dans la formule (I) si simultanément n vaut 2. De tels (co)polymères peuvent en outre comprendre au moins un motif dérivé d'au moins un comonomère copolymérisable avec ledit compose acrylique ou méthacrylique de formule (I) et/ou (II), tel que par exemple :
- un acrylate ou méthacrylate d'alkyle dont le groupe alkyle linéaire ou ramifié, le cas échéant substitué, par exemple par au moins un atome d'halogène comme le chlore ou le fluor et/ou par au moins un groupe hydroxyle, possède de 1 à 20 atomes de carbone,
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-1 naphtalène,
- un nitrile insaturé tel que l'acrylonitrile ou le méthacrylonitrile,
- une maléimide N-substituée telle que la N-éthylmaléimide, la N-isopropylmaléimide, la N-n-butylmaléimide, la N-isobutylmaléimide, la N-terbutylmaléimide, la N-n-octylmaléimide, la N-cyclohexylmaléimide, la N-benzylmaléimide et la N-phénylmaléimide,
- un anhydride d'acide dicarboxylique insaturé tel que l'anhydride maléique, l'anhydride itaconique, l'anhydride citraconique ou l'anhydride tétrahydrophtalique,
- l'acide acrylique ou méthacrylique,
- un acrylate ou méthacrylate de polyol comme les diacrylates et diméthacrylates de l'éthylèneglycol, du propylèneglycol, du 1,3-butanediol, du 1,4-butanediol, du 1,6-hexane-diol, du néopentylglycol, du 1,4-cyclohexane-diol, du 1,4-cyclohexanediméthanol, du 2,2,4-triméthyl-1,3-pentanediol, du 2-éthyl-2-méthyl-1,3-propanediol, du 2,2-diéthyl-1,3-propanediol, du diéthylèneglycol, du dipropylèneglycol, du triéthylèneglycol, du tripropylèneglycol, du tétraéthylèneglycol, du tétrapropylèneglycol, du triméthyloléthane, du triméthylolpropane, du glycérol, du pentaérythritol, les triacrylates et triméthacrylates du triméthyloléthane, du triméthylolpropane, du glycérol, du pentaérythritol, les tétraacrylates et tétraméthacrylates du pentaérythritol, les di(méth)acrylates à hexa(méth)acrylates du dipentaérythritol, les poly(méth)acrylates de polyols mono- ou polyéthoxylés ou mono- ou polypropoxylés tels le triacrylate et le triméthacrylate du triméthylolpropane triéthoxylé, du triméthylolpropane tripropoxylé ; le triacrylate et le triméthacrylate du glycérol tripropoxylé ; le triacrylate, le triméthacrylate, le tétraacrylate et le tétraméthacrylate du pentaérythritol tétraéthoxylé,
- un acrylate ou méthacrylate époxydé choisi parmi le 2-époxyéthylbicyclo[2.2.1]hept-5(6)-yl (méth)acrylate, l'acrylate d'époxydicyclopentyloxyéthyle ainsi que ceux de formule : 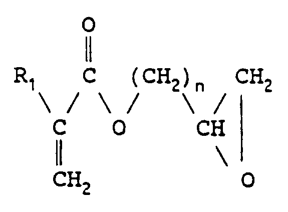 dans laquelle R₁ est choisi parmi l'atome d'hydrogène et le radical méthyle, et n est un nombre entier allant de 1 à 16, ceux de formule : 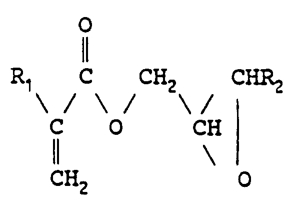 dans laquelle R₁ est choisi parmi l'atome d'hydrogène et le radical méthyle, et R₂ est choisi parmi les radicaux alkyle ayant de 1 à 12 atomes de carbone et les radicaux aryle ayant de 6 à 12 atomes de carbone, et ceux de formules : 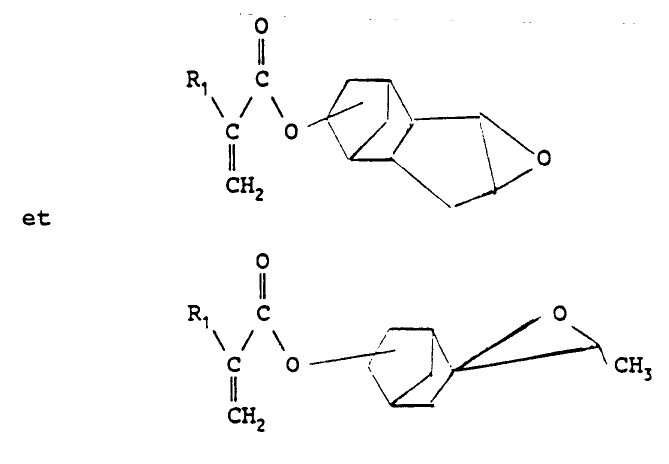 dans lesquelles R₁ est choisi parmi l'atome d'hydrogène et le radical méthyle,
- un acrylamide ou méthacrylamide, un acrylate ou méthacrylate de dialkylaminoalkyle et leurs sels quaternaires,
- l'acrylate et le méthacrylate de (norbornyloxy-2)-2 éthyle et de (diméthanodécahydronaphtyloxy-2)-2 éthyle, et
- des oxazolidones acryliques et méthacryliques choisies parmi celles de formule : 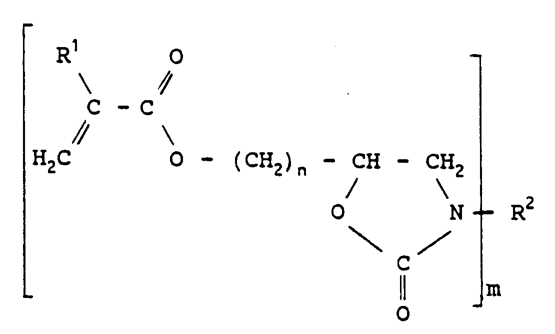 et celles de formule : 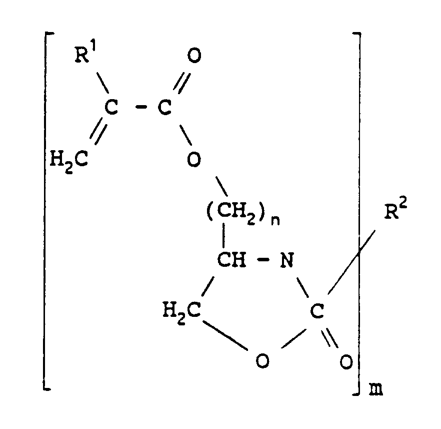 formules dans lesquelles :
   - R¹ est choisi parmi l'atome d'hydrogène et le radical méthyle,
   - n est un nombre entier allant de 1 à 12,
   - m est un nombre entier allant de 1 à 3, et
   - R² est un radical hydrocarboné, alkyle linéaire, ramifié ou cyclique ou bien aromatique, possédant de 5 à 12 atomes de carbone,
   lesdites oxazolidones pouvant être obtenues par réaction, entre 30°C et 90°C, d'un composé portant une fonction (méth)acrylique avec un composé portant au moins une fonction isocyanate,
- des composés acryliques et méthacryliques choisis parmi ceux de formule : 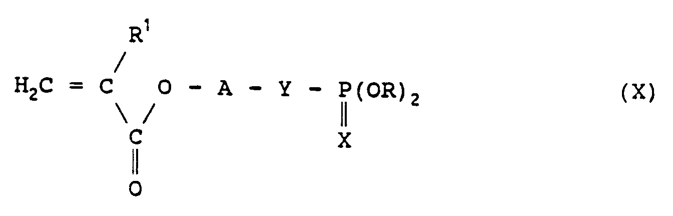 dans laquelle :
   - R¹ est choisi parmi l'atome d'hydrogène et le radical méthyle,
   - A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20,
   - X est choisi parmi les atomes de soufre et d'oxygène,
   - Y est choisi parmi les atomes de soufre et d'oxygène,
   avec la condition que X est un atome de soufre et Y est un atome d'oxygène lorsque A est le radical -(CH₂CH₂O)_{d}-CH₂CH₂-, et
   - R est choisi parmi les radicaux alkyle ayant de 1 à 20 atomes de carbone et les groupes -(CH₂)ₚSR³ dans lesquels p est un nombre entier allant de 3 à 12 et R³ est un radical alkyle ayant de 1 à 20 atomes de carbone,
   ceux de formule : 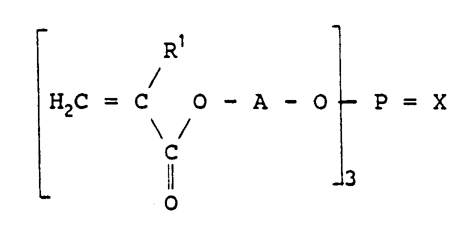 dans laquelle :
   - R¹ est choisi parmi l'atome d'hydrogène et le radical méthyle,
   - A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20, et
   - X est choisi parmi les atomes de soufre et d'oxygène,
   et ceux de formule : 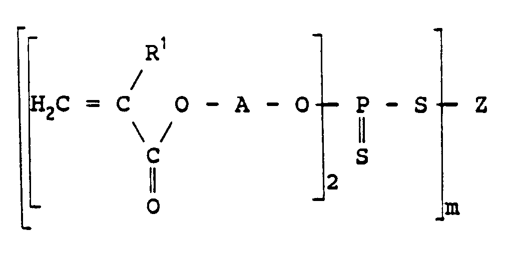 dans laquelle :
   - R¹ est choisi parmi l'atome d'hydrogène et le radical méthyle,
   - A est choisi parmi les radicaux (CH₂)ₙ pour lesquels n est un nombre entier de 2 à 12,
   - m est un nombre entier allant de 1 à 3, et
   - Z est choisi parmi l'atome d'hydrogène, les radicaux R²QH, R² étant un radical alkyle ayant de 2 à 12 atomes de carbone et Q étant choisi parmi les atomes d'oxygène et de soufre, et les atomes des métaux des groupes IA, IIA, IIIA, IB, IIB, VIB, VIIB et VIII de la Classification Périodique,
      avec la condition que Z est choisi parmi l'atome d'hydrogène et les radicaux R²OH lorsque m = 1 et que m est la valence de Z lorsque Z est un métal.
   De tels composés peuvent être préparés par réaction d'un composé acrylique ou méthacrylique de formule : 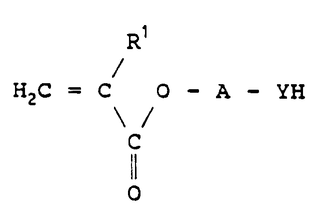 dans laquelle R¹, A et Y ont les mêmes significations que dans la formule (X), avec un composé pentavalent du phosphore, celui-ci pouvant être par exemple un composé de formule PXT₃ dans laquelle X a la même signification que dans la formule (X) et T désigne un atome d'halogène, ou bien un composé phosphoré de formule : 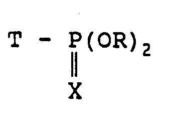 dans laquelle R et X ont les mêmes significations que dans la formule (I) et T désigne un atome d'halogène, ou bien le pentasulfure P₂S₅,
- des composés acryliques et méthacryliques choisis parmi ceux de formule : 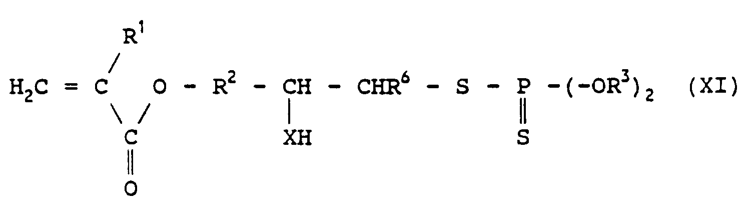 et ceux de formule : 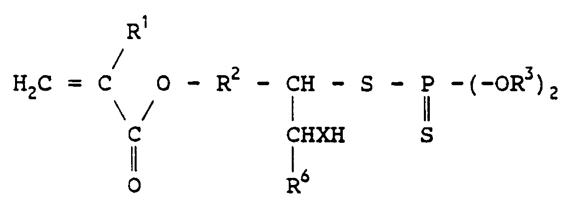 formules dans lesquelles :
   - R¹ est choisi parmi l'atome d'hydrogène et le radical méthyle,
   - X est un hétéroatome choisi parmi l'oxygène et le soufre,
   - R² est choisi parmi les groupes alkylènes linéaires ou ramifiés, cycloalkylènes et hétérocycloalkylènes mono- ou polycycliques, alkylarylènes et arylalkylènes comprenant de 1 à 12 atomes de carbone,
   - R⁶ est choisi parmi l'atome d'hydrogène et les radicaux alkyles et aryles ayant de 1 à 12 atomes de carbone, et
   - R³ est choisi parmi les radicaux alkyles et aryles ayant de 1 à 20 atomes de carbone, les groupes -(CH₂)ₚSR⁴ dans lesquels p est un nombre entier allant de 2 à 12 et R⁴ est un radical alkyle ayant de 1 à 20 atomes de carbone ou bien un groupe cycloalkyle mono- ou polycyclique ayant de 4 à 10 atomes de carbone, chaque cycle dudit groupe comprenant de 4 à 6 chaînons, et les groupes 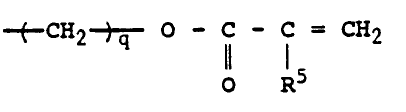 dans lesquels q est un nombre entier allant de 2 à 12 et R⁵ est choisi parmi l'atome d'hydrogène et le radical méthyle. De tels composés peuvent être préparés par réaction d'un époxyde ou épisulfure acrylique ou méthacrylique de formule : 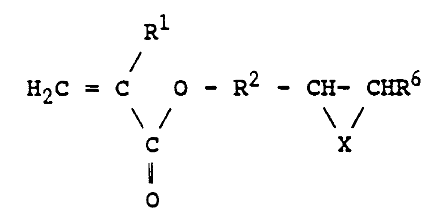 dans laquelle R¹, R², R⁶ et X ont les mêmes significations que dans la formule (XI), avec un composé thiophosphoré de formule :

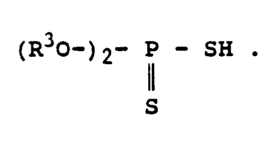

De tels polymères et copolymères sont obtenus en (co)polymérisant au moins un composé acrylique ou méthacrylique de formule (I) et/ou (II) et le cas échéant au moins un comonomère copolymérisable, tel que défini précédemment, en présence d'au moins un initiateur de radicaux libres tel qu'un peroxyde, un hydroperoxyde ou un composé diazo. La (co)polymérisation est généralement effectuée à une température comprise entre 50°C et 120°C environ et en utilisant l'un des monomères comme solvant. Elle peut également s'effectuer en émulsion dans l'eau, à une température comprise entre 50°C et 100°C, en présence d'au moins un agent tensio-actif.

Les exemples suivants illustrent la présente invention sans intention limitative.

### EXEMPLES 1 à 5

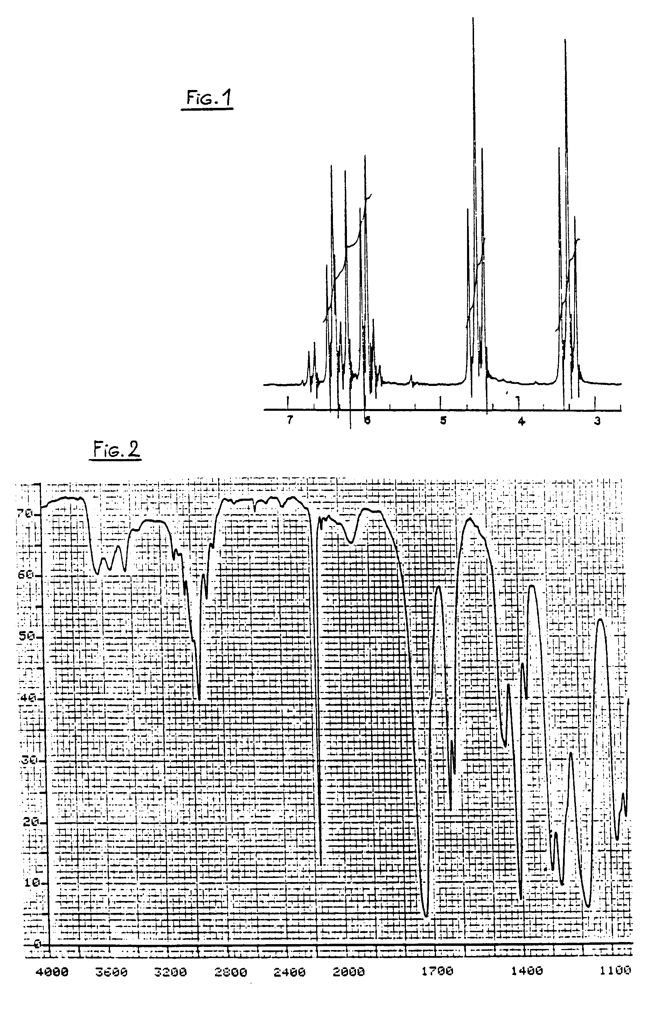

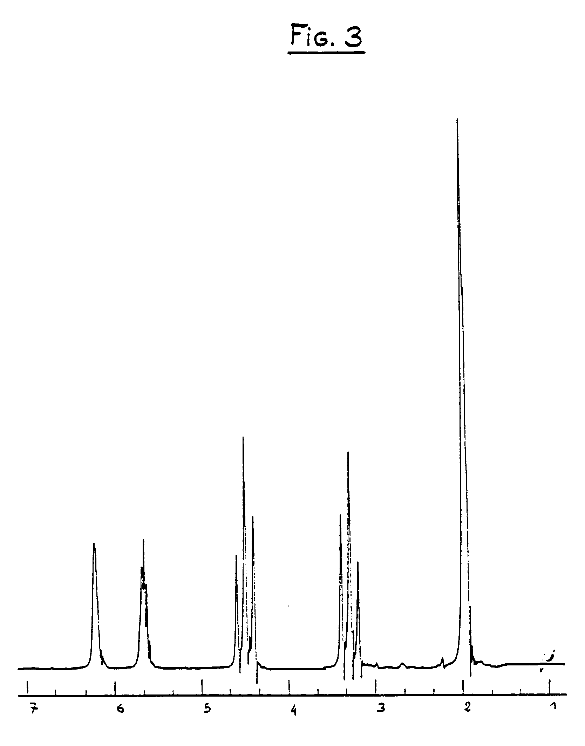

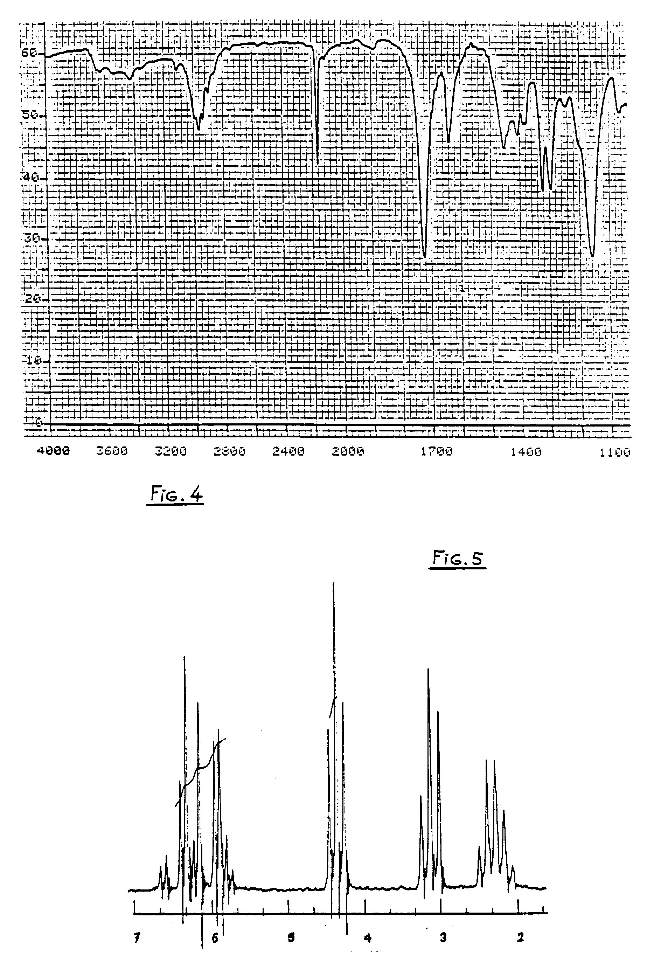

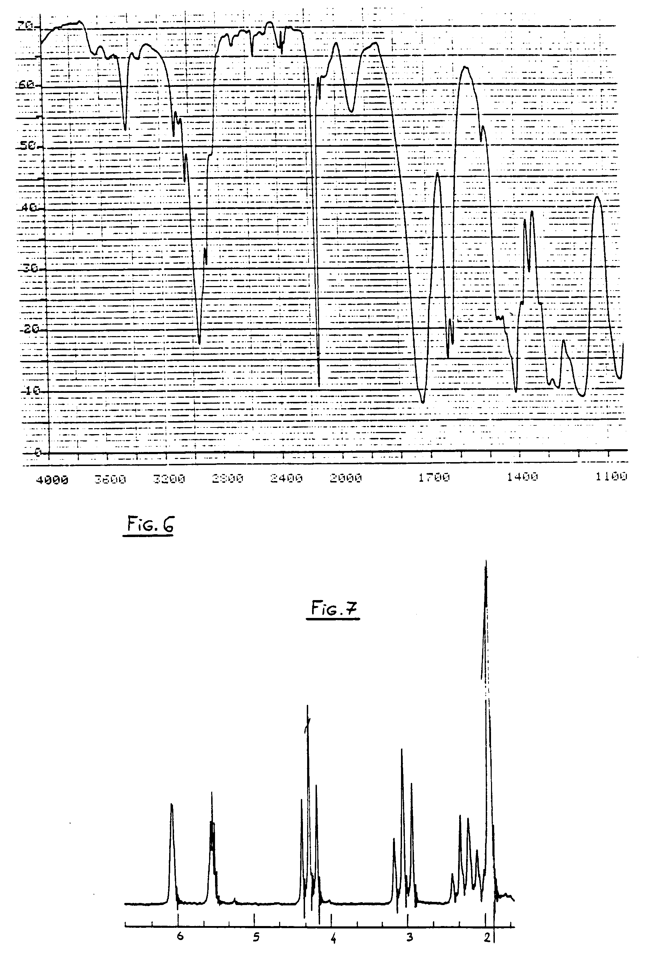

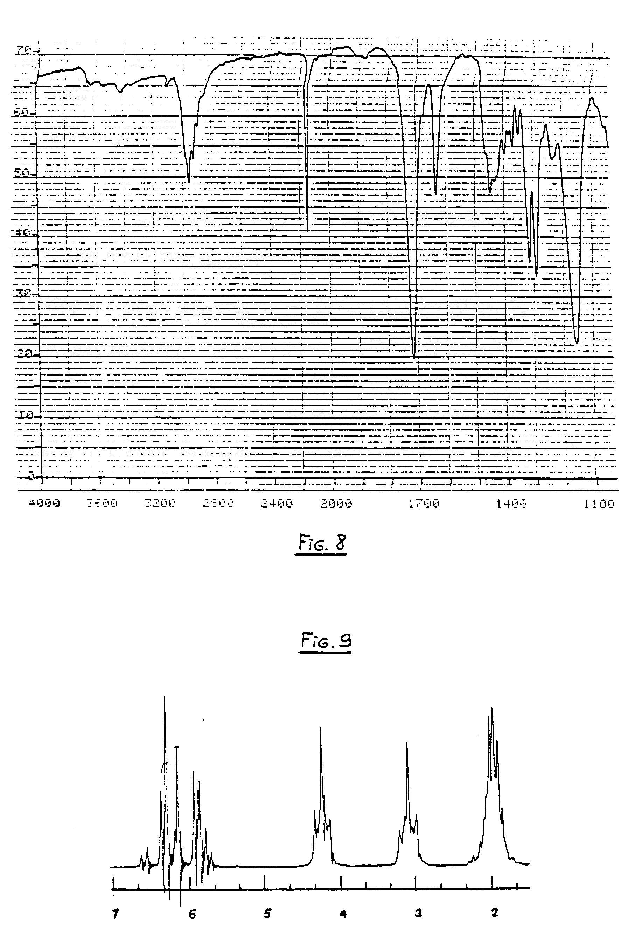

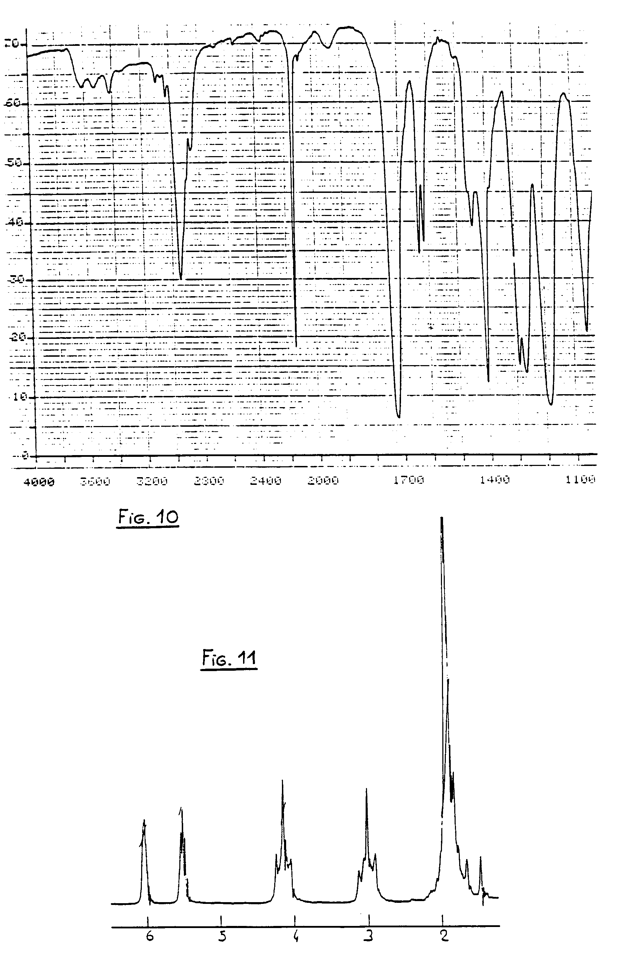

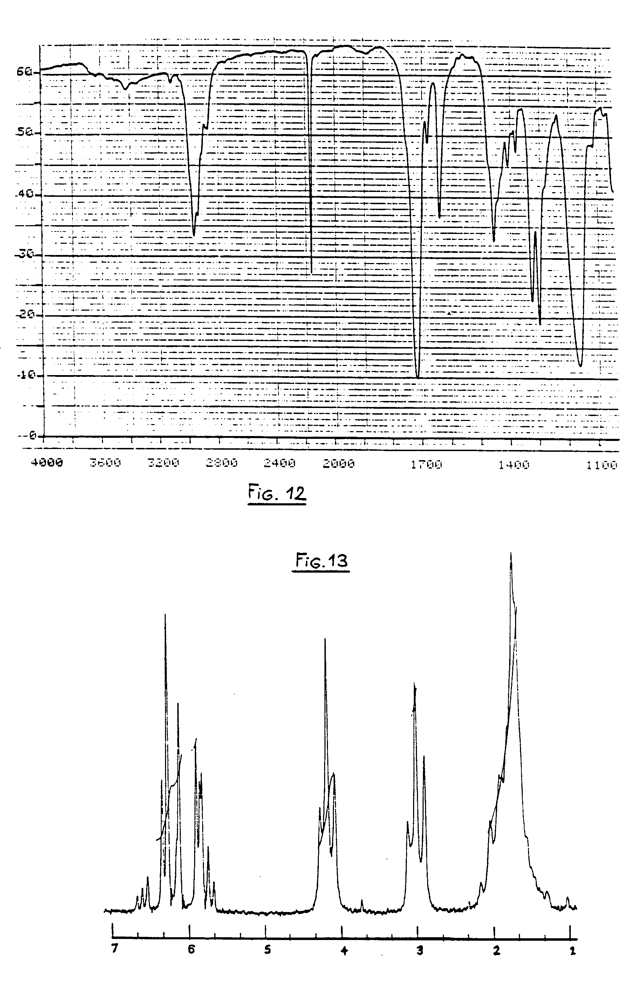

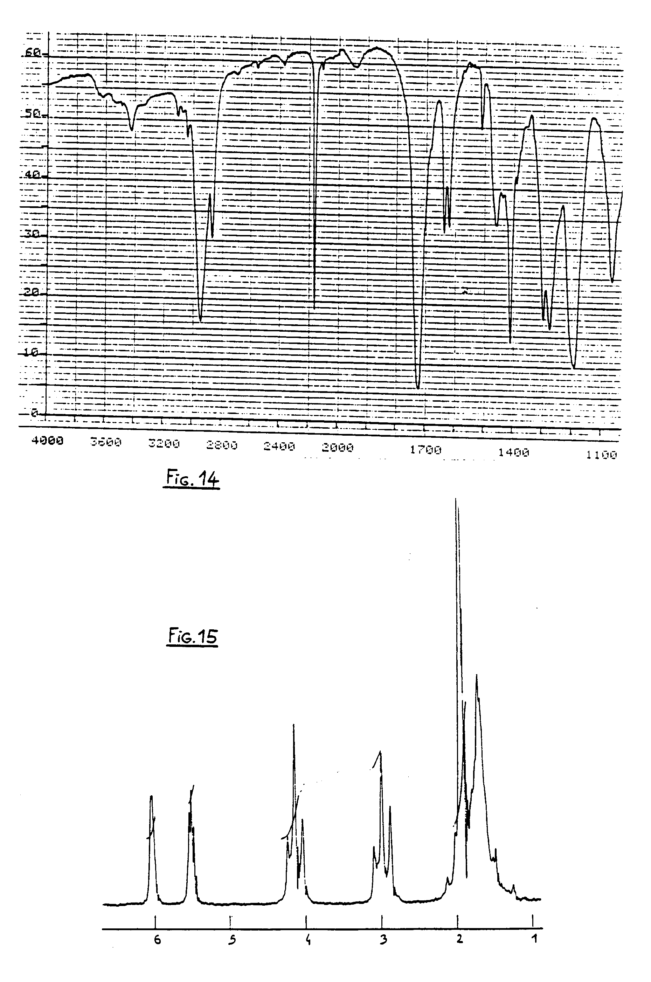

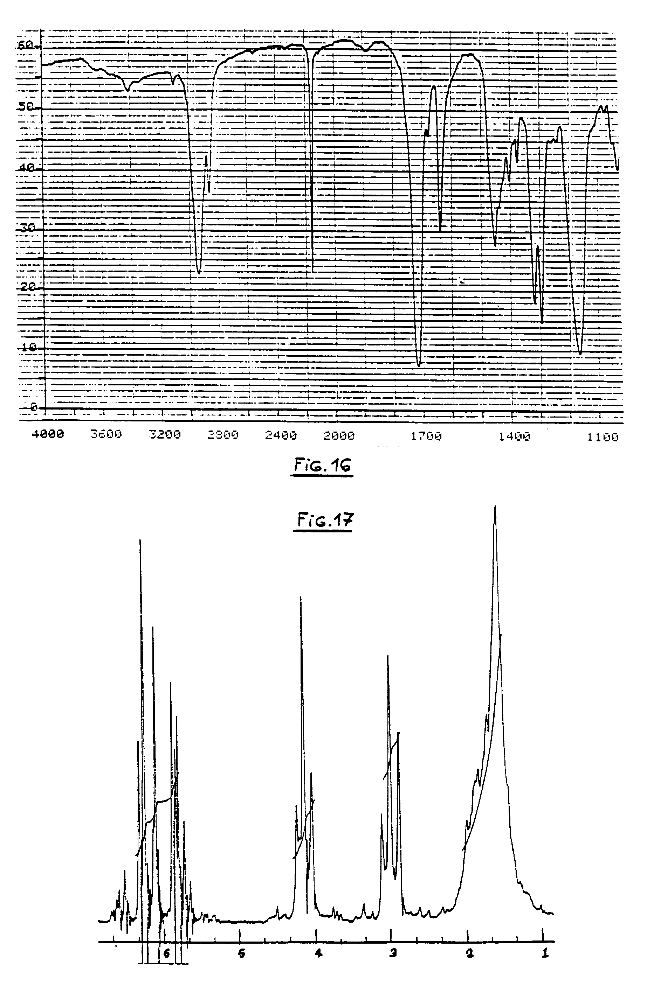

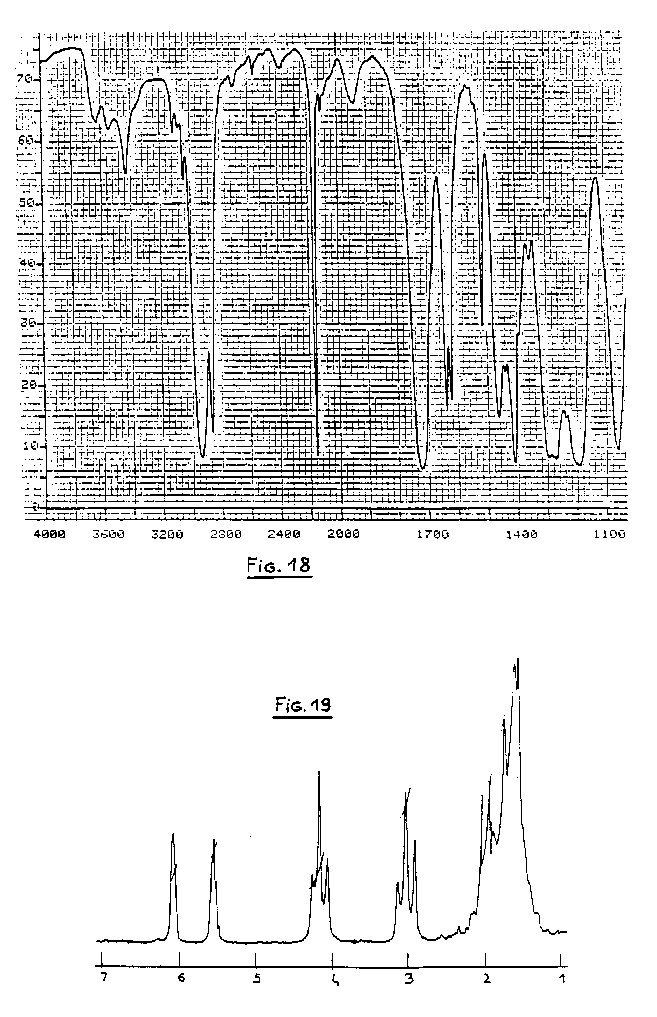

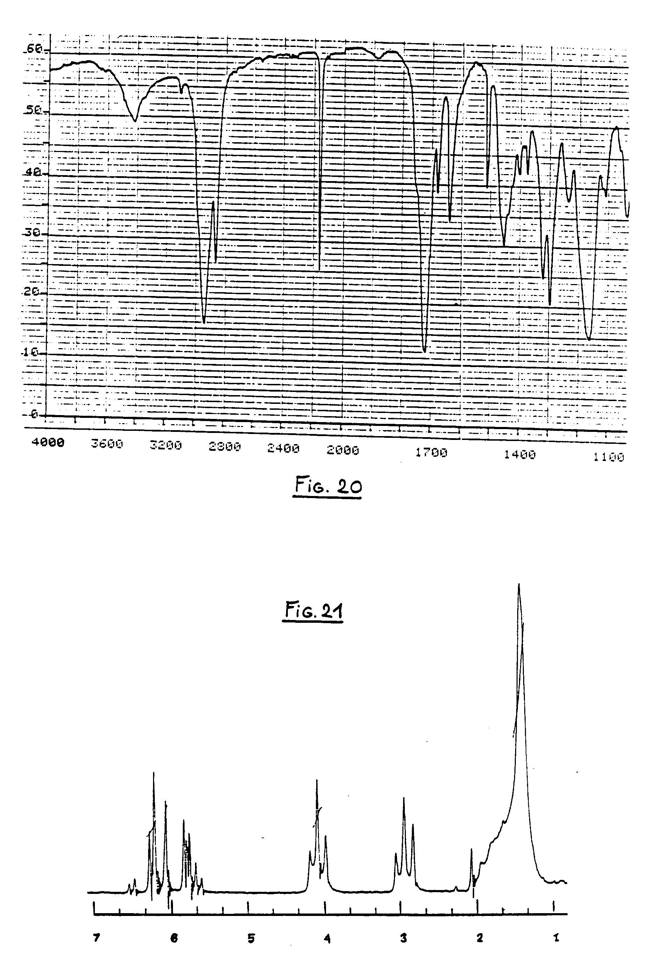

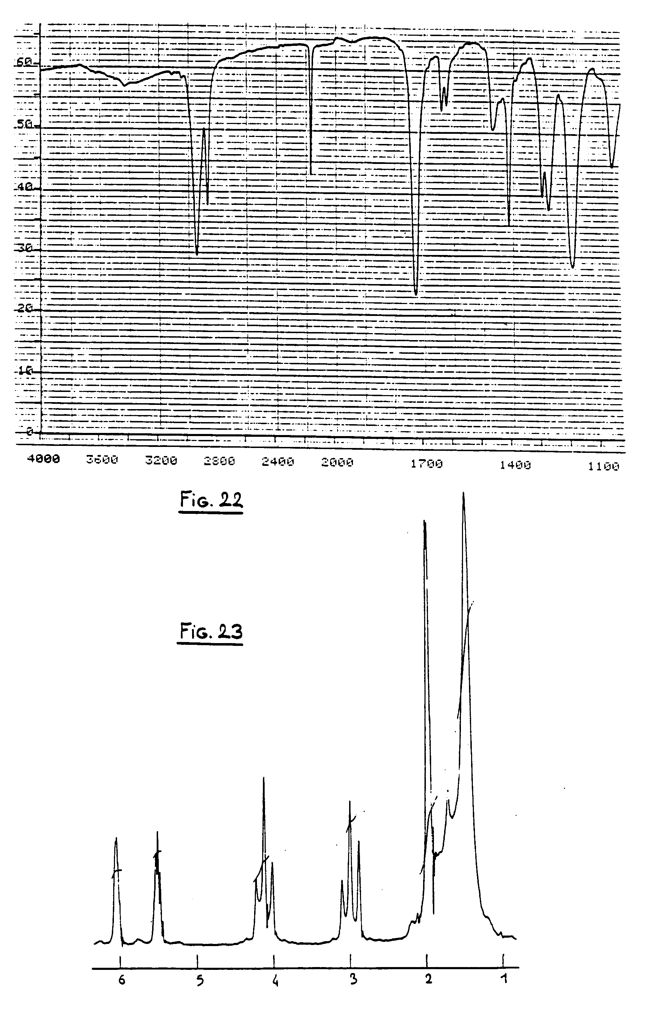

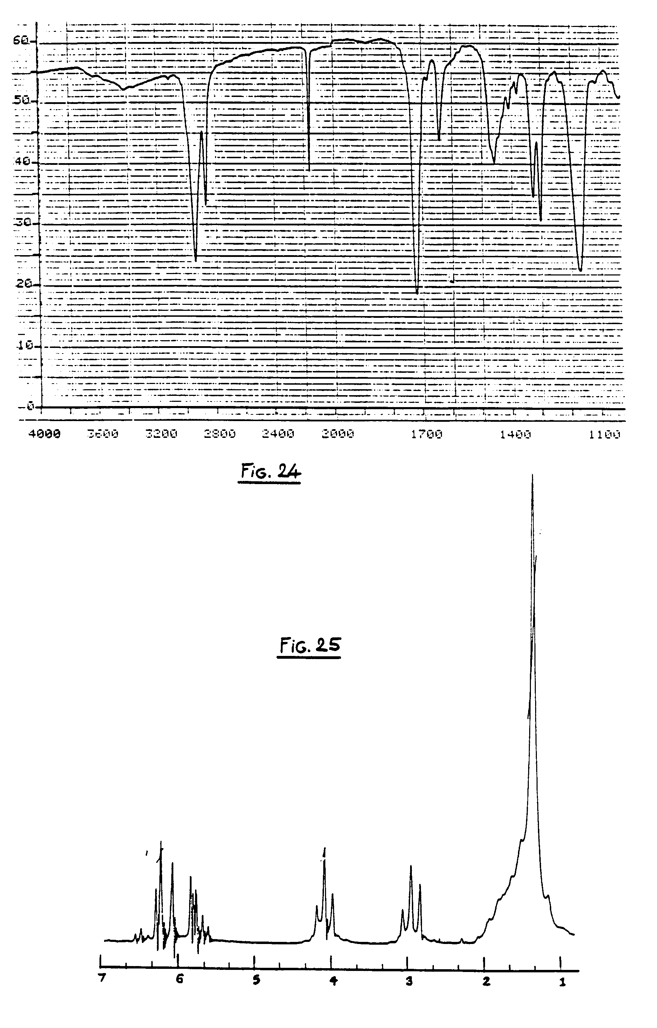

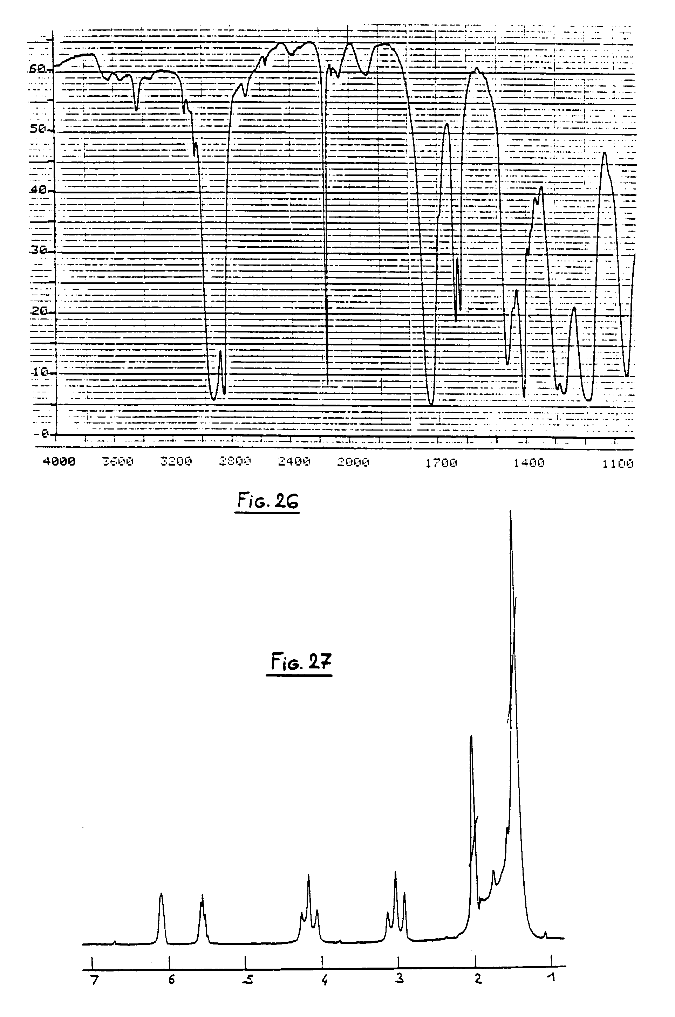

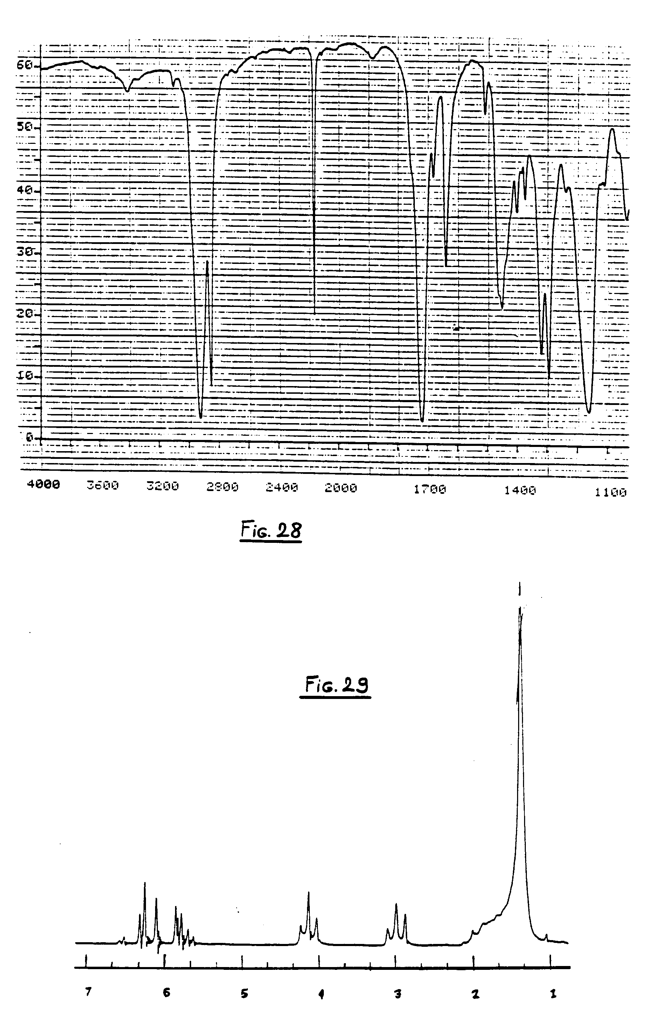

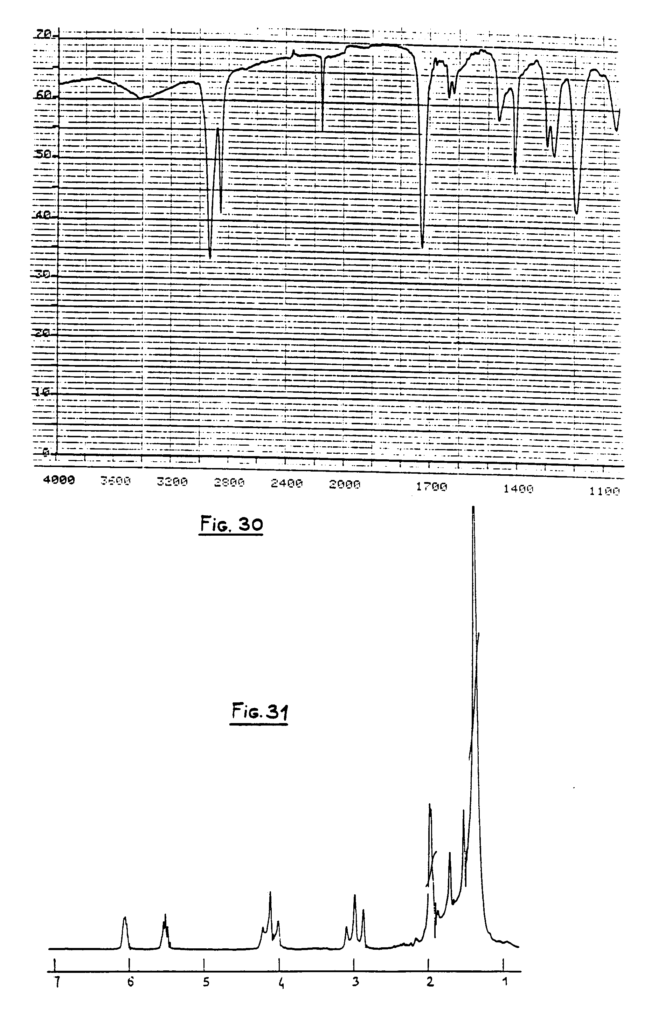

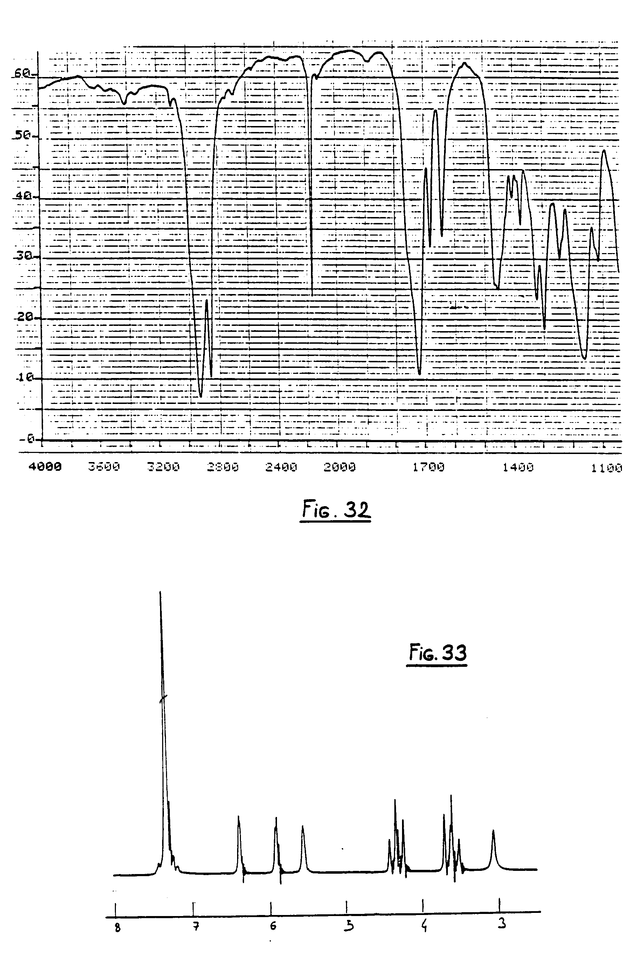

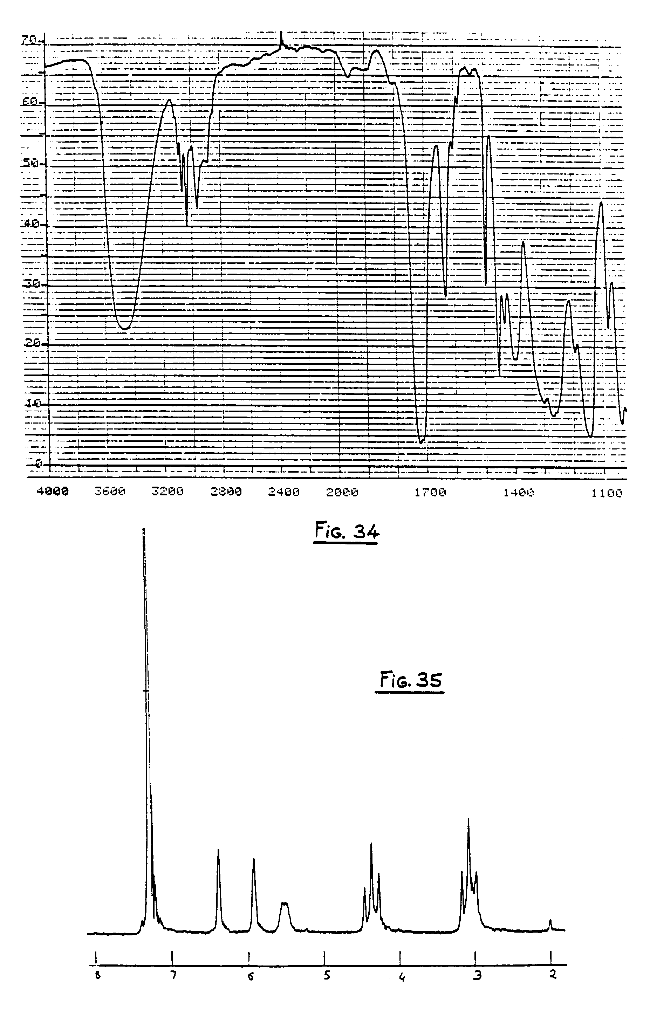

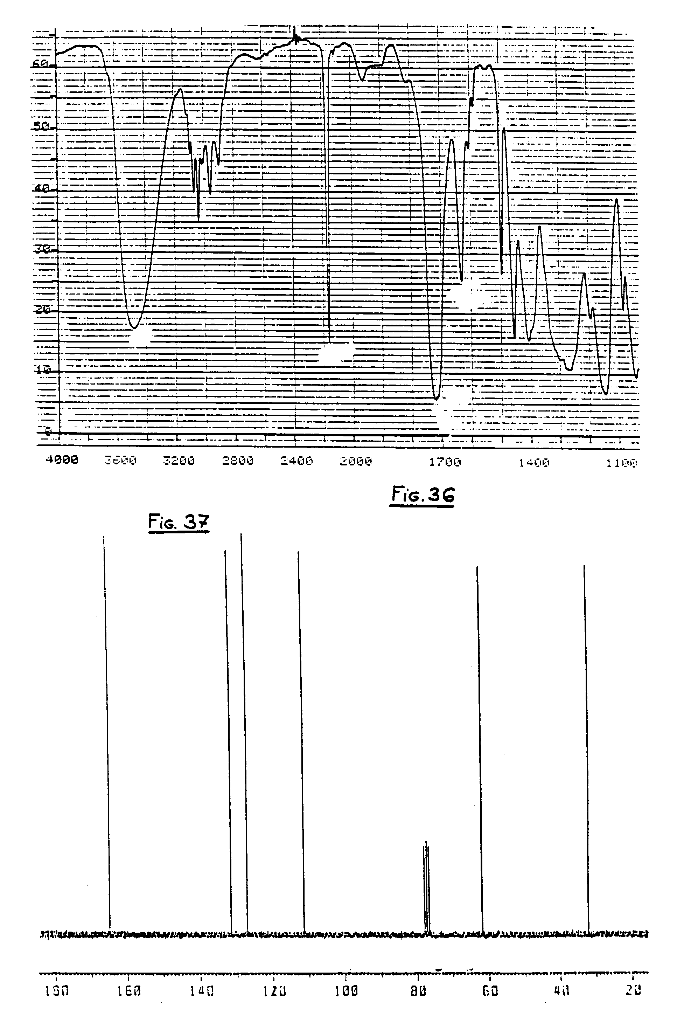

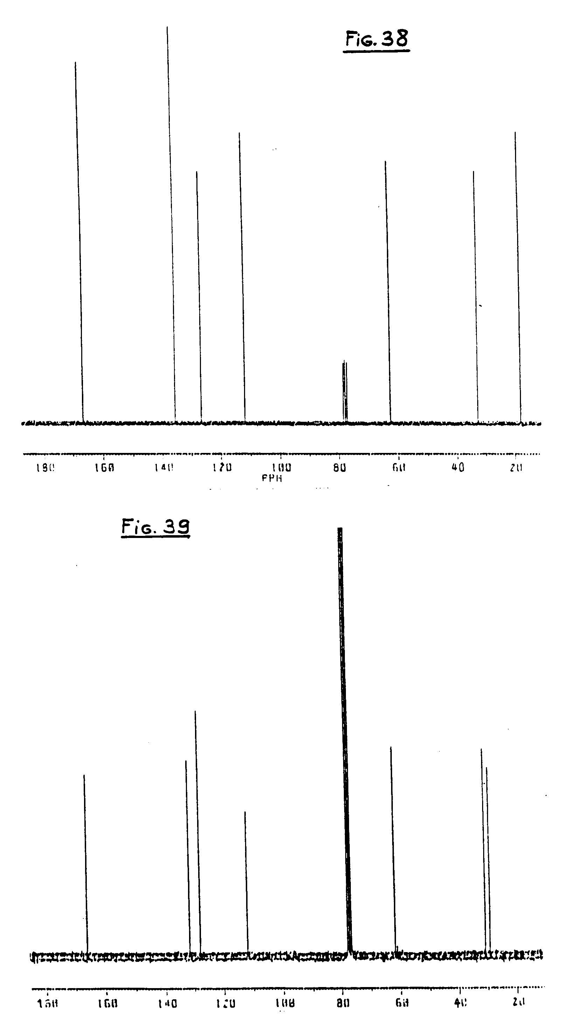

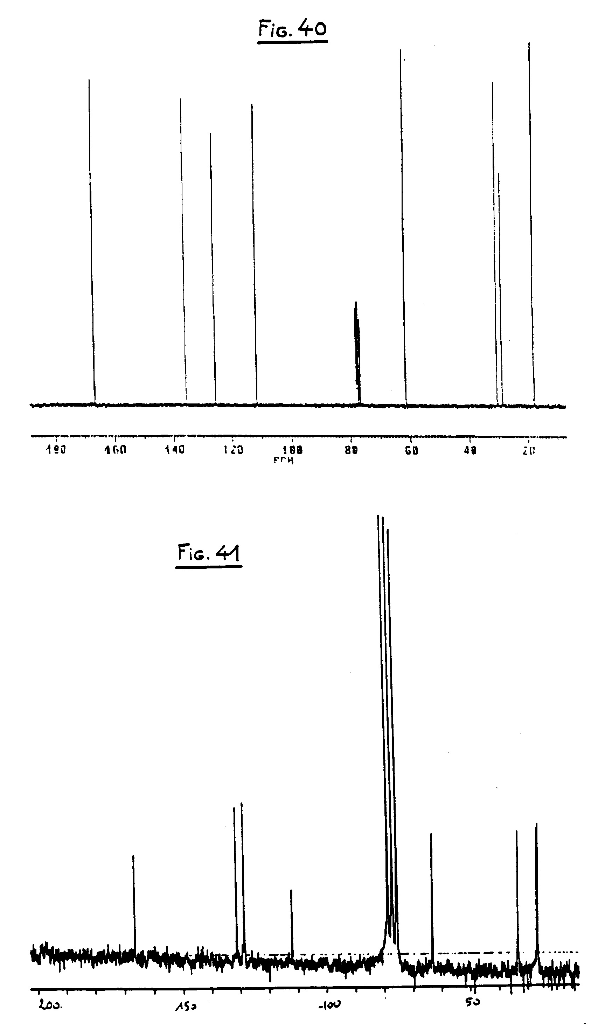

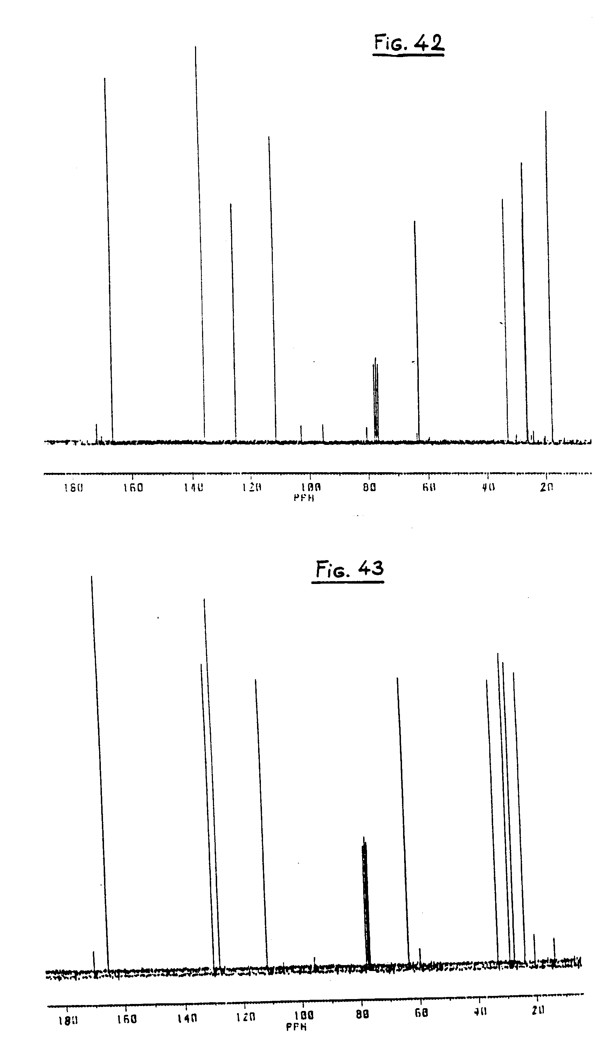

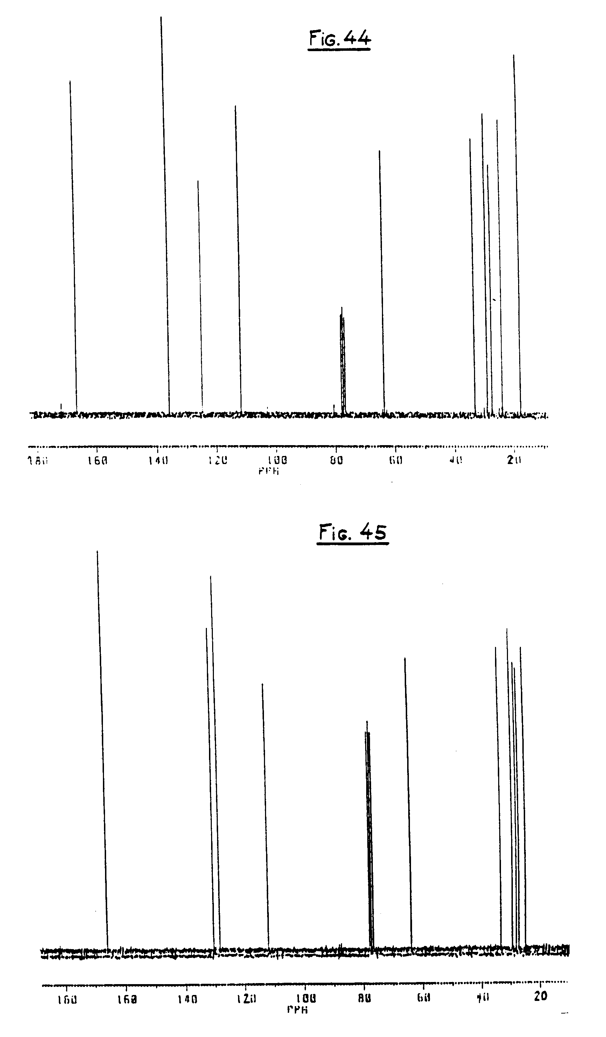

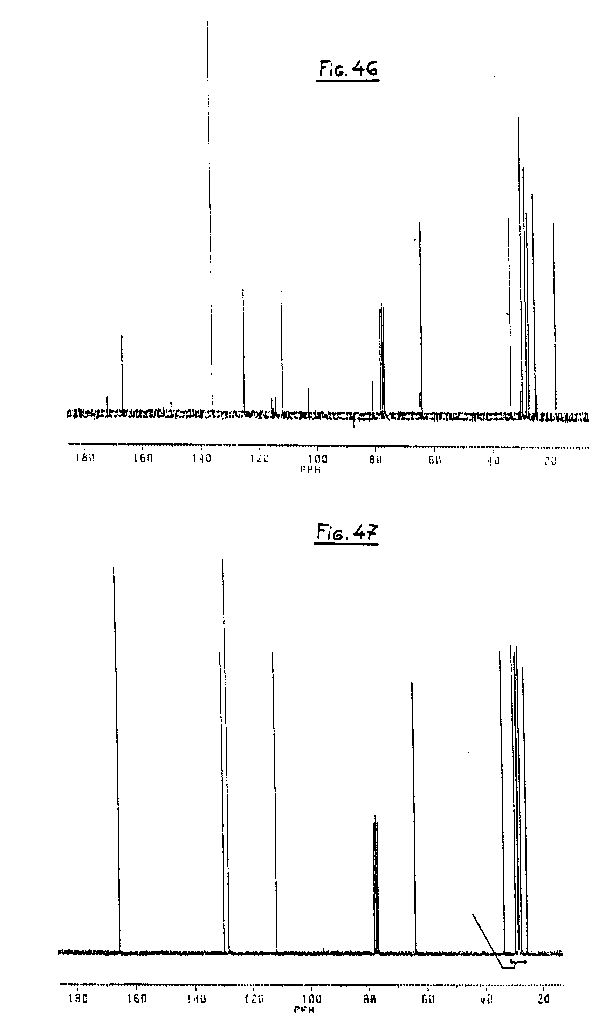

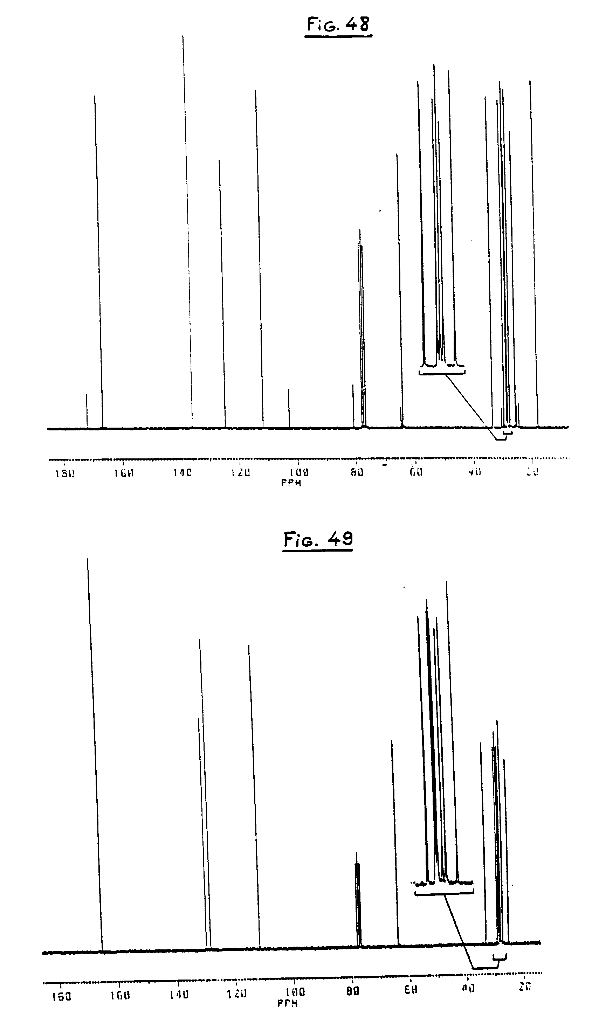

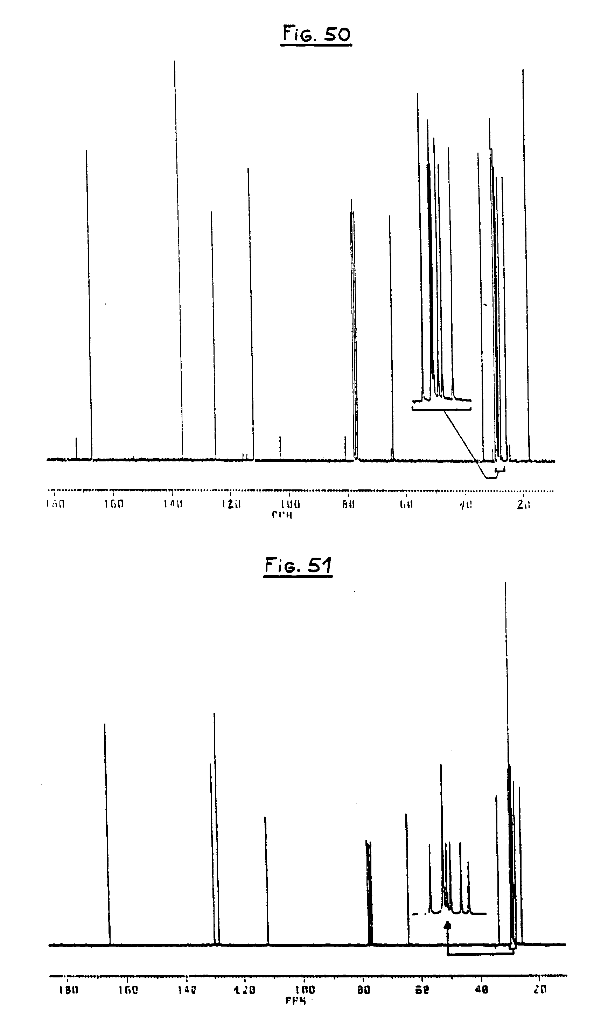

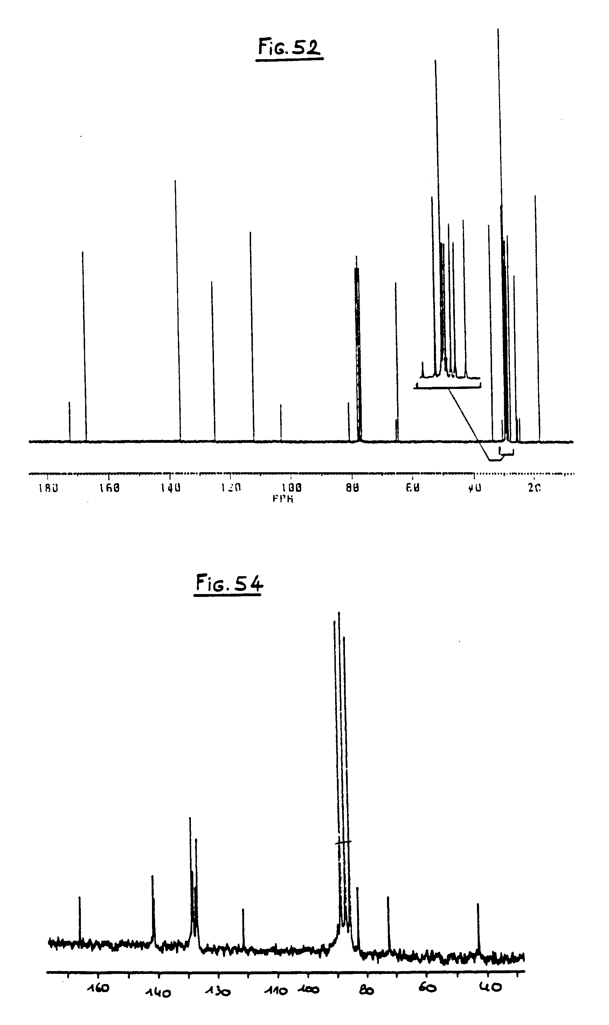

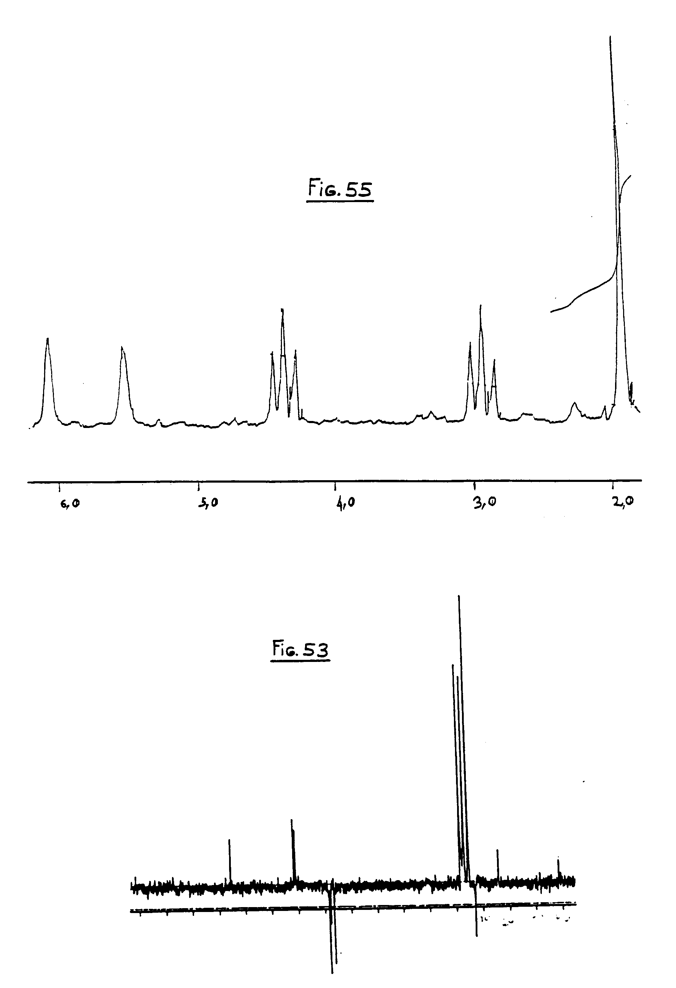

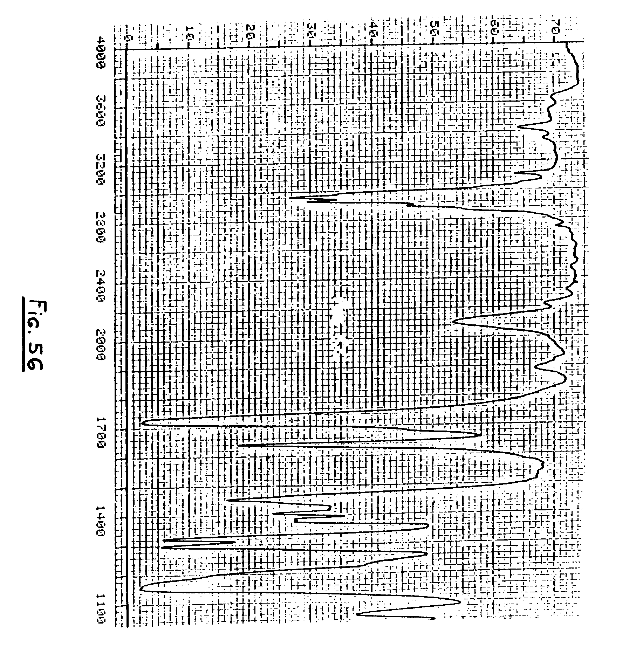

On fait réagir à 65°C dans la méthyléthylcétone le thiocyanate de potassium (2 modes) avec l'acrydate de chloro-2-éthyle (1 mole) en présence d'iodure de tétra-n-butylammonium comme agent de transfert de phase et le cas échéant en présence d'iodure de potassium. Le nombre de modes de chacun des deux derniers composés cités est indiqué dans le Tableau I ci-après, ainsi que la durée de la réaction t (exprimée en heures) et le rendement R en acrylate de thiocyanato-2-éthyle, exprimé en pourcentage et obtenu après isolement et purification sur gel de silice. L'acrylate de thiocyanato-2-éthyle a par ailleurs été identifié par les techniques suivantes :
- résonance magnétique nucléaire du proton, utilisant un spectromètre JEOL PMX60SI : le spectre obtenu comporte deux triplets à 3,25 ppm (2H) et 4,40 ppm (2H) et un multiplet à 6,2 ppm (3H) (Figure 1 en annexe).
- spectrophotométrie infrarouge, utilisant un spectromètre PERKIN ELMER 841 : le spectre obtenu comporte des bandes caractéristiques à 2957, 2157, 1727, 1638, 1620, 1409 et 1183 cm⁻¹ (Figure 2 en annexe).
- résonance magnétique nucléaire du carbone 13, utilisant un spectromètre BRUKER AC 200 : le spectre obtenu est reproduit sur la figure 37 en annexe.

**TABLEAU I**

| Exemple | 1 | 2 | 3 | 4 | 5 |
|---|---|---|---|---|---|
| (C₄H₉)₄NI | 0,1 | 0,2 | 0,2 | 0,2 | 0,3 |
| KI | 0,1 | 0 | 0,1 | 0,5 | 0,1 |
| t | 72 | 120 | 63 | 63 | 48 |
| R | 62 | 81 | 79 | 78 | 76 |

### EXEMPLES 6 à 11

On reproduit la procédure opèratoire de l'Exemple 3 en remplaçant l'iodure de tétra-n-butylammonium par un autre agent de transfert de phase dont la nature est précisée dans le Tableau II ci-après, ainsi que la durée t de la réaction et le rendement R(%).

**TABLEAU II**

| Exemple | Agent de transfert de phase | t | R |
|---|---|---|---|
| 6 | (C₄H₉)₄NHSO₄ | 75 | 55 |
| 7 | (C₄H₉)₄NBr | 75 | 71 |
| 8 | (C₄H₉)₄NCl | 72 | 72 |
| 9 | (C₆H₅)₃CH₃PBr | 84 | 90 |
| 10 | (CH₃)₃C₆H₅NBr | 120 | 73 |
| 11 | CH₃N[(CH₂)₇CH₃]₃Cl | 144 | 52 |

### EXEMPLE 12

On reproduit la procédure opératoire de l'Exemple 7, en remplaçant l'iodure de potassium par une quantité équivalente de bromure de potassium. Après 113 heures de réaction à 65°C, l'acrylate de thiocyanato-2-éthyle est obtenu avec un rendement 78%.

### EXEMPLES 13 à 20

On reproduit la procédure opératoire de l'Exemple 9, en remplaçant la méthyléthylcétone par un autre solvant dont la nature est précisée au Tableau III ci-après et en modifiant corrélativement la température réactionnelle T (exprimée en degré Celsius). Le Tableau III ci-après indique en outre la durée de la réaction et le rendement R (exprimé en pourcentage) en acrylate de thiocyanato-2-éthyle dans chaque cas.

**TABLEAU III**

| Exemple | Solvant | T | t | R |
|---|---|---|---|---|
| 13 | diméthylformamide | 65 | 48 | 68 |
| 14 | éthanol | 65 | 120 | 52 |
| 15 | acétonitrile | 65 | 72 | 76 |
| 16 | benzonitrile | 65 | 120 | 76 |
| 17 | N-N'-diméthylacétamide | 65 | 42 | 90 |
| 18 | toluène | 90 | 72 | 73 |
| 19 | méthylisobutylcétone | 69 | 96 | 70 |
| 20 | acétone | 56 | 168 | 64 |

### EXEMPLES 21 à 35

On reproduit la procédure opératoire de l'Exemple 9 en remplaçant l'acrylate de chloro-2-éthyle par un acrylate ou méthacrylate d'halogéno-n-alkyle de formule (III) en quantité équivalente. On obtient ainsi un acrylate ou méthacrylate de thiocyanato-n-alkyle de formule (I) qui est identifié par les mêmes techniques que celles mentionnées aux Exemples 1 à 5 au sujet de l'acrylate de thiocyanato-2-éthyle. Le Tableau IV ci-après rassemble, en fonction de la valeur de n, de la signification de R et X dans la formule (III), et de la durée t de la réaction (exprimée en heures), le rendement R avec lequel ledit produit est obtenu ainsi que les numéros des figures - en annexe - reproduisant les spectres de résonance magnétique nucléaire du proton (RMN ¹H) et du carbone 13 (RMN ¹³C) et infrarouge (IR) du produit obtenu. Ces spectres présentent des caractères communs et peuvent être décrits comme suit :
- résonance magnétique nucléaire du proton : signal (δ) m, 1H m, 1H t, 2H t, 2H m, 3H (2n-4)H R=CH₃, n=2 6,20 5,65 4,45 3,3 2,0 R=CH₃, n>2 6,05 5,35 4,15 3,0 1,9 1,0-2,0 R=H, n>2 : 5,7-6,7 ppm (m, 3H), 4,15 ppm (t, 2H), 2,95 ppm (t, 2H) et 1,5-2,2 ppm (m, (2n-4)H).
- spectrophotométrie infrarouge : ν en cm⁻¹ R=CH₃ 2158-2154, 1723-1716, 1640-1638 R=H 2157-2154, 1727-1724, 1638-1637, 1622-1619

**TABLEAU IV**

| Exemple | n | R | X | t | R | RMN¹H | IR | RMN ¹³C |
|---|---|---|---|---|---|---|---|---|
| 21 | 2 | CH₃ | Cl | 84 | 76 | 3 | 4 | 38 |
| 22 | 3 | H | Cl | 45 | 92 | 5 | 6 | 39 |
| 23 | 3 | CH₃ | Cl | 26 | 79 | 7 | 8 | 40 |
| 24 | 4 | H | Cl | 31 | 75 | 9 | 10 | 41 |
| 25 | 4 | CH₃ | Cl | 55 | 81 | 11 | 12 | 42 |
| 26 | 5 | H | Br | 2 | 89 | 13 | 14 | 43 |
| 27 | 5 | CH₃ | Br | 2 | 82 | 15 | 16 | 44 |
| 28 | 6 | H | Br | 2 | 82 | 17 | 18 | 45 |
| 29 | 6 | CH₃ | Br | 2 | 90 | 19 | 20 | 46 |
| 30 | 8 | H | Br | 2 | 85 | 21 | 22 | 47 |
| 31 | 8 | CH₃ | Br | 2 | 81 | 23 | 24 | 48 |
| 32 | 10 | H | Br | 2 | 84 | 25 | 26 | 49 |
| 33 | 10 | CH₃ | Br | 2 | 75 | 27 | 28 | 50 |
| 34 | 12 | H | Br | 2 | 95 | 29 | 30 | 51 |
| 35 | 12 | CH₃ | Br | 2 | 91 | 31 | 32 | 52 |

### EXEMPLE 36

On fait réagir pendant 24 heures à la température de 20°C 1 mole d'acrylate de chloro-2-éthyle et 0,7 mole de benzaldéhyde en présence de 0,1 mole de diazabicyclo-[2,2,2]-octane et de 0,1 mole de chlorure de lithium. On obtient alors 0,45 mole d'acrylate de formule : 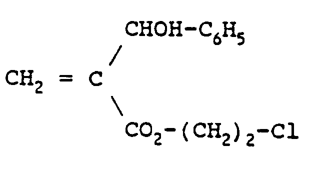
(rendement par rapport au benzaldéhyde = 64%).

Cet acrylate, identifié par les mêmes techniques que celles mentionnées aux Exemples 1 à 5 au sujet de l'acrylate de thiocyanato-2-éthyle, présente les caractéristiques spectrales suivantes :
- résonance magnétique du proton (figure 33 en annexe) :
   7,25 ppm (m, 5H) ; 6,3 ppm (m, 1H) ; 5,9 ppm (m, 1H); 5,45 ppm (s, 1H) ; 4,25 ppm (t, 2H) ; 3,55 ppm (t, 2H) ; 3 ppm (s, 1H),
- spectrophotométrie infrarouge (figure 34 en annexe) :
   3472 cm⁻¹ ; 1721 cm⁻¹ ; 1630 cm⁻¹,
- résonance magnétique du carbone 13 (figure 53 en annexe) :
   165,60 ppm ; 141,73 ppm ; 141,16 ppm ; 128,61 ppm ; 128,37 ppm ; 127,81 ppm ; 126,56 ppm ; 72,92 ppm ; 64,21 ppm ; 41,22 ppm.

### EXEMPLE 37

On fait réagir 0,2 mole de l'acrylate préparé à l'Exemple 36 avec 0,4 mole de thiocyanate de potassium, pendant 168 heures à 65°C dans 200 ml de méthyléthylcétone, en présence de 0,04 mole de bromure de méthyltriphénylphosphonium et de 0,02 mole d'iodure de potassium. On obtient alors 0,148 mole d'acrylate de formule : 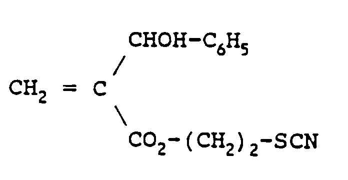
(rendement par rapport à l'acrylate = 74%).

Cet acrylate, identifié par les mêmes techniques que celles mentionnées aux Exemples 1 à 5 au sujet de l'acrylate de thiocyanato-2-éthyle, présente les caractéristiques spectrales suivantes :
- résonance magnétique du proton (figure 35 en annexe) :
   7,25 ppm (m, 5H) ; 6,35 ppm (m, 1H) ; 5,9 ppm (m, 1H); 5,5 ppm (m, 1H) ; 4,35 ppm (t, 2H) ; 3,05 ppm (t, 2H) ; 3 ppm (s, 1H),
- spectrophotométrie infrarouge (figure 36 en annexe) :
   3484 cm⁻¹ ; 2157 cm⁻¹ ; 1721 cm⁻¹ ; 1630 cm⁻¹,
- résonance magnétique du carbone 13 (figure 54 en annexe) :
   165,48 ppm ; 141,44 ppm ; 141,15 ppm ; 128,44 ppm ; 127,92 ppm ; 127,16 ppm ; 126,63 ppm ; 111,24 ppm ; 72,77 ppm ; 62,29 ppm ; 32,50 ppm.

### EXEMPLE 38

On fait réagir 0,5 mole de l'acrylate de thiocyanato-2-éthyle obtenu conformément à l'Exemple 9 avec 0,35 mole de benzaldéhyde, pendant 48 heures à la température de 20°C , en présence de 0,05 mole de diazabicyclo-[2,2,2]-octane et de 0,05 mole de chlorure de lithium. On obtient alors 0,231 mole du même produit, identifié par ses caractéristiques spectrales infrarouge et de résonance magnétique nucléaire, que celui obtenu à l'Exemple 37. (Rendement par rapport au benzaldéhyde : 66%).

### EXEMPLE 39

On fait réagir 0,3 mole du méthacrylate de thiocyanato-2-éthyle obtenu conformément à l'Exemple 21 avec 0,1 mole d'hydrure de sodium, à 55°C pendant 24 heures dans 400 ml de toluène. En suivant l'avancement de la réaction par chromatographie en phase gazeuse, on observe alors l'obtention d'un mélange constitué à 75% de méthacrylate de thiocyanato-2-éthyle et à 25% de son trimère de formule (II), celui-ci étant identifié de la manière suivante :
a) Résonance magnétique nucléaire du proton :
   A l'exception des pics à 6,2 ppm (m, 1H), à 5,65 ppm (m, 1H), à 4,5 ppm (t, 2H) et à 2 ppm (m, 3H) dont les déplacements chimiques sont conservés, le spectre (figure 55 en annexe) indique la présence d'un triplet à 3,05 ppm (2H).
b) Spectrophotométrie infrarouge (figure 56 en annexe) :
   Le spectre indique la présence d'une nouvelle bande caractéristique à 2096 cm⁻¹.

### EXEMPLE 40

On reproduit la procédure opératoire de l'Exemple 39 à l'exception de la quantité d'hydrure de sodium, portée à 0,3 mole, et de la durée de la réaction, portée à 72 heures. On obtient alors un mélange de 47% du méthacrylate de thiocyanato-2-éthyle et de 53% de son trimère de formule (II) identifié comme précédemment.

### EXEMPLE 41

On reproduit la procédure opératoire de l'Exemple 39 à l'exception de la quantité d'hydrure de sodium, portée à 0,9 mole, et de la durée de la réaction, portée à 72 heures. On obtient alors un mélange de 80% du méthacrylate de thiocyanato-2-éthyle et de 20% de son trimère de formule (II), identifié comme précédemment.

## Revendications

1. Composés acryliques et méthacryliques choisis parmi ceux de formule : 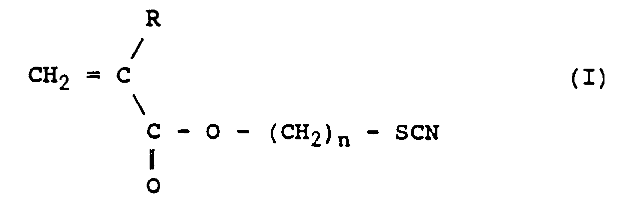 et ceux de formule 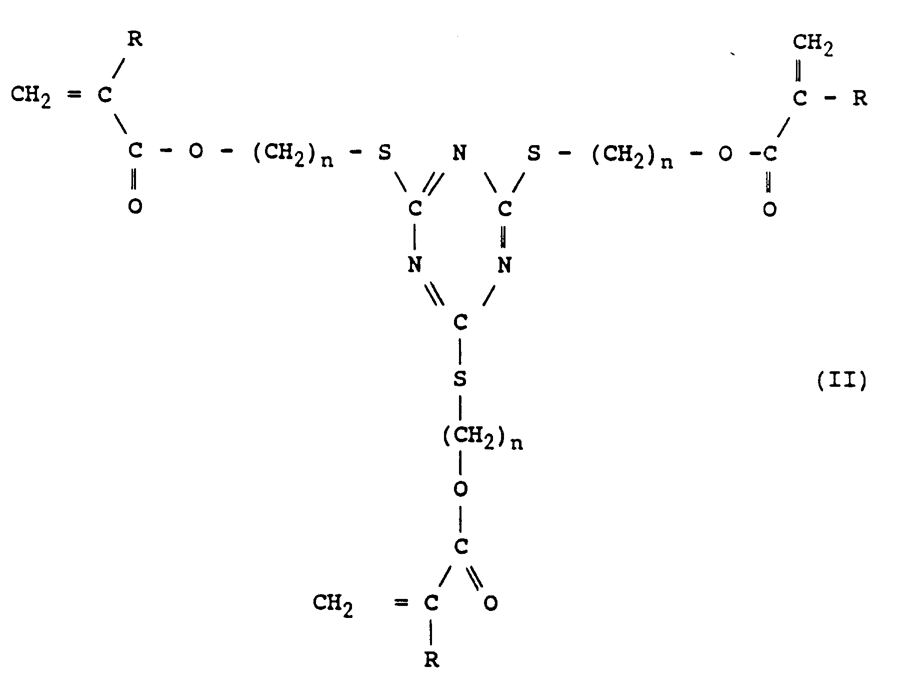 dans lesquelles :
- n est un nombre entier allant de 2 à 20, et
- R est choisi parmi l'atome d'hydrogène, le radical méthyle et les radicaux CHOHR' dans lesquels R' est choisi parmi les radicaux alkyles linéaires ou ramifiés le cas échéant porteurs de substituants hydroxyles, alcoxy ou esters, les radicaux alcényles linéaires, ramifiés ou cycliques, les radicaux aryles le cas échéant porteurs de substituants halogénés, nitrés ou alcoxy, les radicaux hétérocycliques insaturés, les radicaux alkylaryles et arylalkyles,
R ne pouvant représenter méthyle dans la formule (I) si simultanément n vaut 2 ou 4.

2. Procédé de préparation d'un composé acrylique et méthacrylique de formule (I) dans laquelle R est choisi parmi l'atome d'hydrogène et le radical méthyle, et n est un nombre entier allant de 2 à 20, consistant à faire réagir un acrylate ou méthacrylate d'halogéno-n-alkyle de formule : 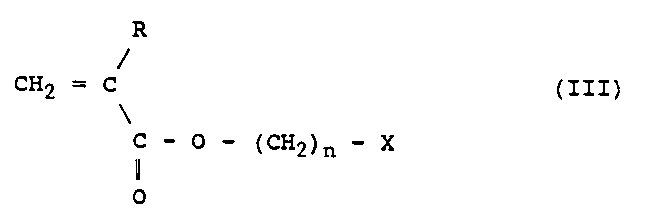 dans laquelle n est tel que défini ci-dessus et X est un atome d'halogène, avec un sel de thiocyanate dans un solvant organique et en présence d'une quantité efficace d'au moins un agent de transfert de phase.

3. Procédé de préparation d'un composé acrylique de formule (I) dans laquelle R est choisi parmi les radicaux CHOHR' et n est un nombre entier allant de 2 à 20, comprenant dans une première étape la préparation d'un composé acrylique de formule (I) dans laquelle R est l'atome d'hydrogène conformément au procédé selon la revendication 2 puis dans une seconde étape la réaction dudit composé acrylique avec un aldéhyde de formule R'CHO en présence d'une quantité efficace d'au moins un catalyseur de fonctionnalisation.

4. Procédé de préparation d'un composé acrylique de formule (I) dans laquelle R est choisi parmi les radicaux CHOHR' et n est un nombre entier allant de 2 à 20, comprenant dans une première étape la réaction d'un acrylate d'halogéno-n-alkyle de formule : 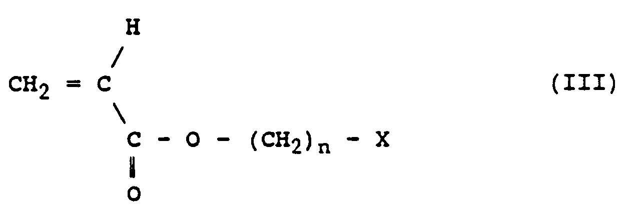 dans laquelle n est tel que défini ci-dessus et X est un atome d'halogène, avec un aldéhyde de formule R'CHO en présence d'une quantité efficace d'au moins un catalyseur de fonctionnalisation pour obtenir un intermédiaire de formule : 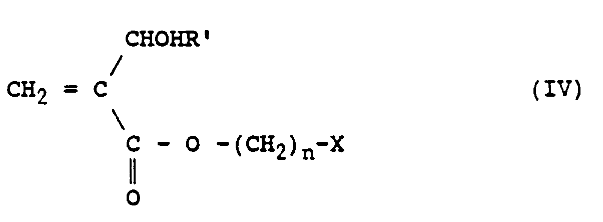 puis dans une seconde étape la réaction dudit intermédiaire avec au moins un sel de thiocyanate dans un solvant organique et en présence d'une quantité efficace d'au moins un agent de transfert de phase.

5. Procédé de préparation d'un composé acrylique ou méthacrylique de formule (I) selon l'une des revendications 2 à 4, caractérisé en ce que le sel de thiocyanate est choisi parmi les thiocyanates de métal alcalin et le thiocyanate d'ammonium.

6. Procédé de préparation d'un composé acrylique ou méthacrylique de formule (I) selon l'une des revendications 2 à 5, caractérisé en ce que le solvant organique de la réaction entre le sel de thiocyanate et l'acrylate de formule (III) ou bien l'intermédiaire de formule (IV) est choisi parmi les cétones, les amides, les hydrocarbures cyclaniques et aromatiques, les alcools et les nitriles.

7. Procédé de préparation d'un composé acrylique ou méthacrylique de formule (I) selon l'une des revendications 2 à 6, caractérisé en ce que l'agent de transfert de phase est choisi parmi les sels d'ammonium quaternaires, les sels de phosphonium quaternaires et les sels d'arsonium quaternaires.

8. Procédé de préparation d'un composé acrylique ou méthacrylique de formule (I) selon l'une des revendications 3 à 7, caractérisé en ce que le catalyseur de fonctionnalisation est choisi parmi les amines tertiaires cycliques ayant au moins un atome d'azote commun à trois cycles.

9. Procédé de préparation d'un composé acrylique ou méthacrylique de formule (II) comprenant la réaction d'un composé acrylique ou méthacrylique de formule (I) avec au moins un hydrure de métal alcalin, en présence d'un solvant organique.

10. En tant qu'intermédiaire dans la préparation d'un composé acrylique ou méthacrylique de formule (I) selon la revendication 1, R étant choisi parmi les radicaux CHOHR', un composé acrylique de formule : 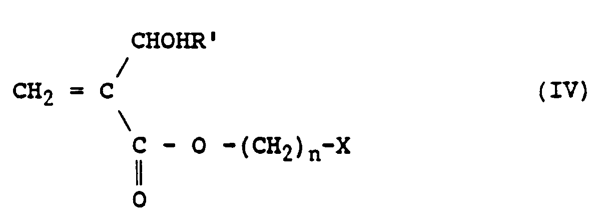 dans laquelle n et R' ont la même signification que dans la revendication 1 et X désigne un atome d'halogène.

11. Polymère comprenant au moins un motif dérivé d'un composé acrylique ou méthacrylique représenté par la formule (I) telle que définie à la revendication 1, dans laquelle :
- n est un nombre entier allant de 2 à 20, et
- R est choisi parmi l'atome d'hydrogène, le radical méthyle et les radicaux CHOHR' dans lesquels R' est choisi parmi les radicaux alkyles linéaires ou ramifiés le cas échéant porteurs de substituants hydroxyles, alcoxy ou esters, les radicaux alcényles linéaires, ramifiés ou cycliques, les radicaux aryles le cas échéant porteurs de substituants halogénés, nitrés ou alcoxy, les radicaux hétérocycliques insaturés, les radicaux alkylaryles et arylalkyles,
R ne pouvant représenter méthyle si simultanément n vaut 2 ;
et/ou par la formule (II) telle que définie à la revendication 1.

## Claims

1. Acrylic and methacrylic compounds chosen from those of formula: 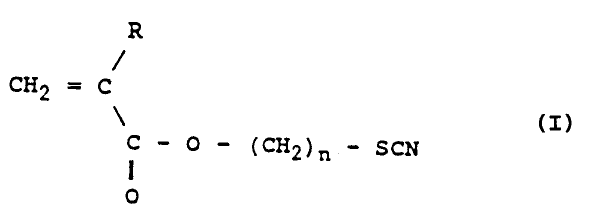 and those of formula 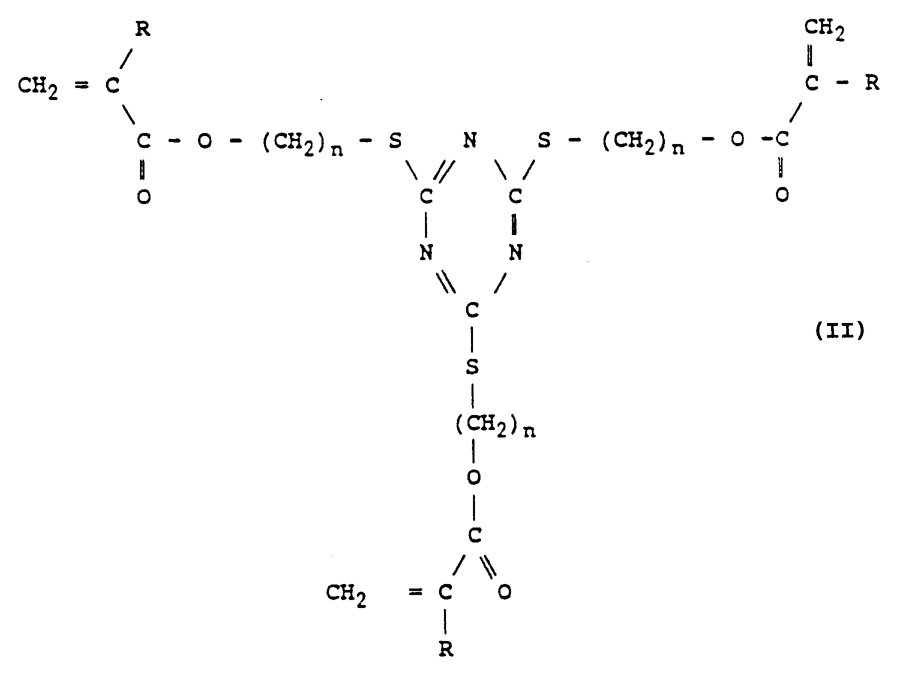 in which formulae:
- n is an integer ranging from 2 to 20, and
- R is chosen from a hydrogen atom, a methyl radical and the radicals CHOHR' in which R' is chosen from straight-chain or branched alkyl radicals, which if necessary carry hydroxyl, alkoxy or ester substituents, straight-chain, branched or cyclic alkenyl radicals, aryl radicals, which if necessary carry halogenated, nitro or alkoxy substituents, unsaturated heterocyclic radicals and alkylaryl and arylalkyl radicals,
R not being able to represent methyl in the formula (I) if n at the same time is 2 or 4.

2. Process for the preparation of an acrylic or methacrylic compound of formula (I) in which R is chosen from a hydrogen atom and a methyl radical and n is an integer ranging from 2 to 20, consisting in reacting a halogeno-n-alkyl acrylate or methacrylate of formula: 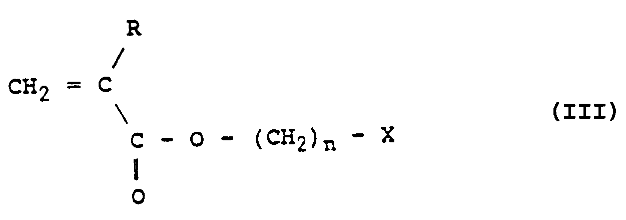 in which n is as defined above and X is a halogen atom, with a thiocyanate salt in an organic solvent and in the presence of an effective amount of at least one phase transfer agent.

3. Process for the preparation of an acrylic compound of formula (I) in which R is chosen from the radicals CHOHR' and n is an integer ranging from 2 to 20, comprising, in a first step, the preparation of an acrylic compound of formula (I) in which R is a hydrogen atom in accordance with the process according to Claim 2 and then, in a second step, the reaction of the said acrylic compound with an aldehyde of formula R'CHO in the presence of an effective amount of at least one functionalisation catalyst.

4. Process for the preparation of an acrylic compound of formula (I) in which R is chosen from the radicals CHOHR' and n is an integer ranging from 2 to 20, comprising, in a first step, the reaction of a halogeno-n-alkyl acrylate of formula 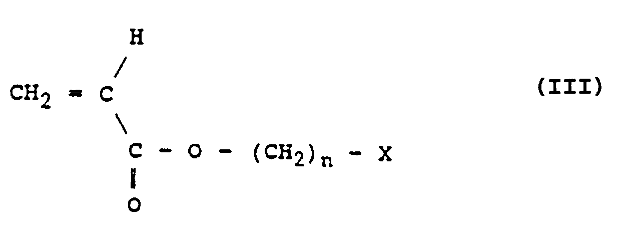 in which n is as defined above and X is a halogen atom, with an aldehyde of formula R'CHO in the presence of an effective amount of at least one functionalisation catalyst in order to obtain an intermediate of formula: 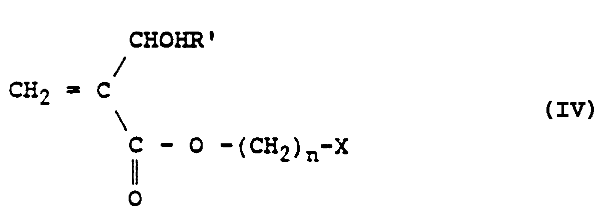 and then, in a second step, the reaction of the said intermediate with at least one thiocyanate salt in an organic solvent and in the presence of an effective amount of at least one phase transfer agent.

5. Process for the preparation of an acrylic or methacrylic compound of formula (I) according to one of Claims 2 to 4, characterised in that the thiocyanate salt is chosen from alkali metal thiocyanates and ammonium thiocyanate.

6. Process for the preparation of an acrylic or methacrylic compound of formula (I) according to one of Claims 2 to 5, characterised in that the organic solvent for the reaction between the thiocyanate salt and the acrylate of formula (III) or the intermediate of formula (IV) is chosen from ketones, amides, cycloalkane and aromatic hydrocarbons, alcohols and nitriles.

7. Process for the preparation of an acrylic or methacrylic compound of formula (I) according to one of Claims 2 to 6, characterised in that the phase transfer agent is chosen from quaternary ammonium salts, quaternary phosphonium salts and quaternary arsonium salts.

8. Process for the preparation of an acrylic or methacrylic compound of formula (I) according to one of claims 2 to 7 characterized in that functionalisation catalyst is choosen from cyclic tertiary amines having at least one nitrogen atom common to three rings.

9. Process for the preparation of an acrylic or methacrylic compound of formula (II) comprising the reaction of an acrylic or methacrylic compound of formula (I) with at least one alkali metal hydride in the presence of an organic solvent.

10. An acrylic compound of formula: 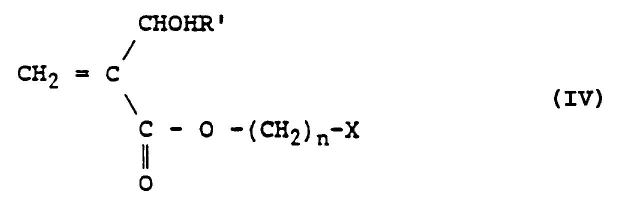 in which n and R' have the same meaning as in Claim 1 and X denotes a halogen atom,
as an intermediate in the preparation of an acrylic or methacrylic compound of formula (I) according to Claim 1, R being chosen from the radicals CHOHR'.

11. Polymer comprising at least one unit derived from an acrylic or methacrylic compound represented by the formula (I) as defined in Claim 1, in which:
- n is an integer ranging from 2 to 20, and
- R is chosen from a hydrogen atom, a methyl radical and the radicals CHOHR' in which R' is chosen from straight-chain or branched alkyl radicals, which if necessary carry hydroxyl, alkoxy or ester substituents, straight-chain, branched or cyclic alkenyl radicals, aryl radicals, which if necessary carry halogenated, nitro or alkoxy substituents, unsaturated heterocyclic radicals and alkylaryl and arylalkyl radicals,
R not being able to represent methyl if n at the same time is 2;
and/or by the formula (II) as defined in Claim 1.

## Patentansprüche

1. Acryl- und Methacrylverbindungen, ausgewählt aus jenen der Formel 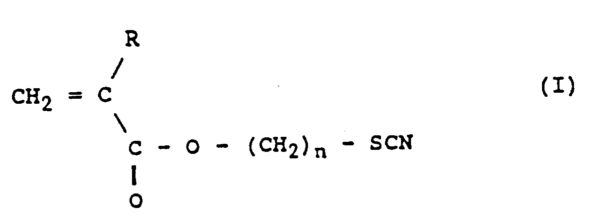 und jenen der Formel 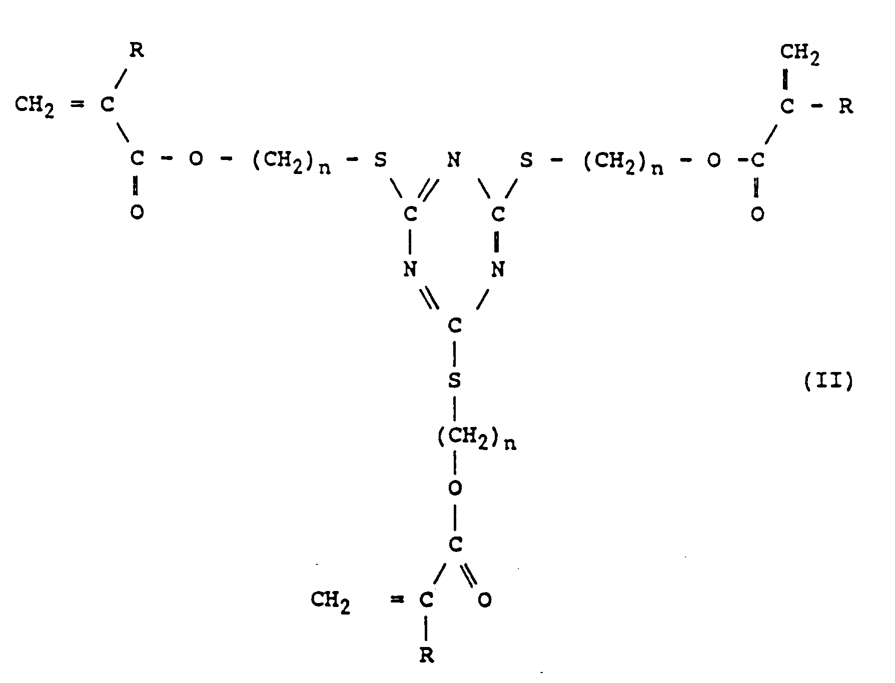 in welchen Formeln
- n eine ganze Zahl von 2 bis 20 ist, und
- R ausgewählt ist aus der Gruppe bestehend aus dem Wasserstoffatom, dem Methylrest und den Resten CHOHR', in denen R' ausgewählt ist aus den linearen oder verzweigten Alkylresten, die gegebenenfalls Träger von Hydroxyl-, Alkoxy- oder Estersubstituenten sind, den linearen, verzweigten oder cyclischen Alkenylresten, den Arylresten, die gegebenenfalls Träger von halogenierten Substituenten, nitrierten Substituenten oder Alkoxysubstituenten sind, den ungesättigten heterocyclischen Resten, den Alkylaryl- und Arylalkylresten,
wobei R in der Formel (I) nicht Methyl darstellen kann, wenn gleichzeitig n den Wert 2 oder 4 hat.

2. Verfahren zur Herstellung einer Acryl- oder Methacrylverbindung der Formel (I), in der R ausgewählt ist aus der Gruppe bestehend aus dem Wasserstoffatom und dem Methylrest, und n eine ganze Zahl von 2 bis 20 ist, welches Verfahren darin besteht, ein Acrylat oder Methacrylat von Halogen-n-alkyl der Formel 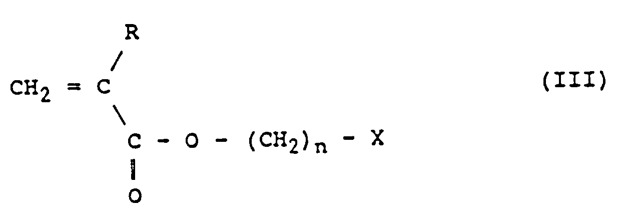 in der n die oben angegebene Bedeutung hat und X ein Halogenatom ist, mit einem Thiocyanatsalz in einem organischen Lösungsmittel und in Gegenwart einer wirksamen Menge mindestens eines Phasenübertragungsmittels reagieren zu lassen.

3. Verfahren zur Herstellung einer Acrylverbindung der Formel (I), in der R ausgewählt ist aus den Resten CHOHR' und n eine ganze Zahl von 2 bis 20 ist, welches Verfahren in einer ersten Stufe die Herstellung einer Acrylverbindung der Formel (I), in der R das Wasserstoffatom ist, nach dem Verfahren nach Anspruch 2 und dann in einer zweiten Stufe die Reaktion der genannten Acrylverbindung mit einem Aldehyd der Formel R'CHO in Gegenwart einer wirksamen Menge mindestens eines Funktionalisierungskatalysators umfaßt.

4. Verfahren zur Herstellung einer Acrylverbindung der Formel (I), in der R ausgewählt ist aus den Resten CHOHR' und n eine ganze Zahl von 2 bis 20 ist, welches Verfahren in einer ersten Stufe die Reaktion eines Acrylats von Halogen-n-alkyl der Formel 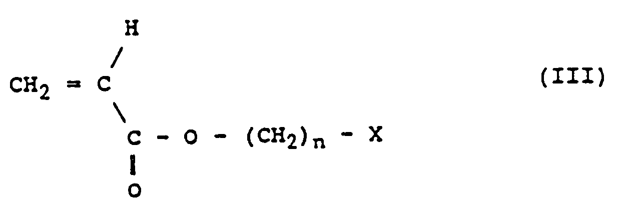 in der n die oben angegebene Bedeutung hat und X ein Halogenatom ist, mit einem Aldehyd der Formel R'CHO in Gegenwart einer wirksamen Menge mindestens eines Funktionalisierungskatalysators, um ein Zwischenprodukt mit der Formel 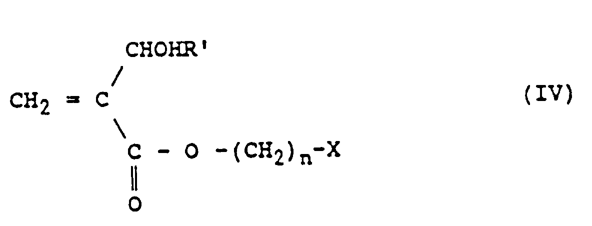 zu erhalten, und in einer zweiten Stufe die Reaktion des genannten Zwischenprodukts mit mindestens einem Thiocyanatsalz in einem organischen Lösungsmittel und in Gegenwart einer wirksamen Menge mindestens eines Phasenübertragungsmittels umfaßt.

5. Verfahren zur Herstellung einer Acryl- oder Methacrylverbindung der Formel (I) nach einem der Ansprüche 2 bis 4, dadurch gekennzeichnet, daß das Thiocyanatsalz ausgewählt wird aus der Gruppe bestehend aus den Alkalimetallthiocyanaten und Ammoniumthiocyanat.

6. Verfahren zur Herstellung einer Acryl- oder Methacrylverbindung der Formel (I) nach einem der Ansprüche 2 bis 5, dadurch gekennzeichnet, daß das organische Lösungsmittel der Reaktion zwischen dem Thiocyanat und dem Acrylat der Formel (III) oder auch dem Zwischenprodukt der Formel (IV) ausgewählt wird aus den Ketonen, den Amiden, den cyclanischen und aromatischen Kohlenwasserstoffen, den Alkoholen und den Nitrilen.

7. Verfahren zur Herstellung einer Acryl- oder Methacrylverbindung der Formel (I) nach einem der Ansprüche 2 bis 6, dadurch gekennzeichnet, daß das Phasenübertragungsmittel ausgewählt wird aus den quartären Ammoniumsalzen, den quartären Phosphoniumsalzen und den quartären Arsoniumsalzen.

8. Verfahren zur Herstellung einer Acryl- oder Methacrylverbindung der Formel (I) nach einem der Ansprüche 3 bis 7, dadurch gekennzeichnet, daß der Funktionalisierungskatalysator ausgewählt wird aus den cyclischen tertiären Aminen mit mindestens einem drei Ringen gemeinsamen Stickstoffatom.

9. Verfahren zur Herstellung einer Acryl- oder Methacrylverbindung der Formel (II), umfassend die Reaktion einer Acryl- oder Methacrylverbindung der Formel (I) mit mindestens einem Alkalimetallhydrid in Gegenwart eines organischen Lösungsmittels.

10. Acrylverbindung als Zwischenprodukt bei der Herstellung einer Acryl- oder Methacrylverbindung der Formel (I) nach Anspruch 1, wobei R ausgewählt ist aus den Resten CHOHR', mit der Formel 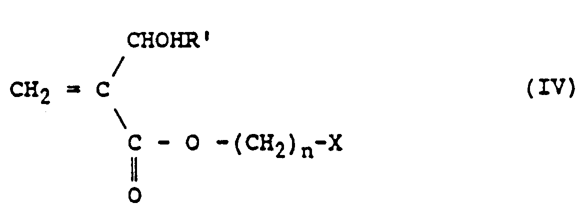 in der n und R' dieselbe Bedeutung haben wie in Anspruch 1 und X ein Halogenatom bezeichnet.

11. Polymer enthaltend mindestens eine Einheit, die abstammt von einer Acryl- oder Methacrylverbindung, die repräsentiert wird durch die in Anspruch 1 angegebene Formel (I), in der
- n eine ganze Zahl von 2 bis 20 ist, und
- R ausgewählt ist aus der Gruppe bestehend aus dem Wasserstoffatom, dem Methylrest und den Resten CHOHR', in denen R' ausgewählt ist aus den linearen oder verzweigten Alkylresten, die gegebenenfalls Träger von Hydroxyl-, Alkoxy- oder Estersubstituenten sind, den linearen, verzweigten oder cyclischen Alkenylresten, den Arylresten, die gegebenenfalls Träger von halogenierten Substituenten, nitrierten Substituenten oder Alkoxysubstituenten sind, den ungesättigten heterocyclischen Resten, den Alkylaryl- und Arylalkylresten,
wobei R in der Formel (I) nicht Methyl darstellen kann, wenn gleichzeitig n den Wert 2 hat, und/oder durch die in Anspruch 1 angegebene Formel (II).
